# EUROPÄISCHE PATENTSCHRIFT

(11) **EP 4 110 223 B1**
(45) Veröffentlichungstag und Bekanntmachung des Hinweises auf die Patenterteilung: **20.12.2023**
(21) Anmeldenummer: 22713593.6
(22) Anmeldetag: 08.03.2022
(51) Int. Cl.: A61B 50/20, A61B 50/33, A61B 50/34

(54) **AUFNAHME-EXPANDERHÜLSE SOWIE WERKZEUGSET**
EXPANDABLE HOLDER SLEEVE, AND TOOL SET
MANCHON DE SUPPORT EXTENSIBLE ET ENSEMBLE D'OUTILS

(30) Priorität: 12.03.2021 DE 102021106109
(43) Veröffentlichungstag der Anmeldung: 04.01.2023
(73) Patentinhaber: Aesculap AG, 78532 Tuttlingen (DE)
(72) Erfinder: AY, Eda, 88512 Mengen (DE)
(74) Vertreter: Winter, Brandl - Partnerschaft mbB
(86) Internationale Anmeldenummer: PCT/EP2022/055849
(87) Internationale Veröffentlichungsnummer: WO 2022/189410

(56) Entgegenhaltungen:
- EP-B1- 0 263 011
- WO-A1-2020/163481
- DE-A1-102004 053 355
- US-A1- 2013 064 709
- US-A1- 2017 143 449

## Beschreibung

### Technisches Gebiet

Die vorliegende Offenbarung betrifft eine Aufnahme-Expanderhülse eines Werkzeugsets zur steckgelagerten Aufnahme chirurgischer Werkzeuge sowie ein Werkzeugset mit der Aufnahme-Expanderhülse und einer Halterungsvorrichtung.

### Hintergrund der Erfindung

In der modernen Chirurgie kommt eine Vielzahl von verschiedenen Instrumentenköpfen/Werkzeugen wie z.B. Bohr- oder Fräswerkzeuge (mit Werkzeugschaft) zum Einsatz, die jeweils auf/in ein einen Instrumentenantrieb/Motor aufweisendes Handstück abnehmbar auf-/eingesteckt werden können. Dies erlaubt dem Operateur, während des Betriebs des Handstücks den auf dem / in das Handstück auf-/eingesteckten Instrumentenschaft mit distalem Instrumentenkopf/Effektor je nach Anforderung der Anwendung zu wechseln. Um dem Operateur hierbei einen möglichst einfachen Zugriff auf diese Instrumentenköpfe (nachstehend nur noch als Werkzeuge bezeichnet) zu ermöglichen, werden die Werkzeuge gewöhnlich in einem chirurgischen Werkzeugset geordnet aufbewahrt, wie dies beispielsweise auch aus dem Handwerk beispielsweise für Bohrersets bekannt ist. Hierbei werden, um Beschädigungen der Werkzeuge zu vermeiden und ein rasches Auffinden eines benötigten Werkzeugs zu gewährleisten, die Werkzeuge jedoch nicht lose aufbewahrt, sondern in Aufnahme-Expanderhülsen in fixierender/eingespannter Weise aufgenommen, die wiederum in einer Lochmatrix des Werkzeugsets (im einfachsten Fall eine Lochplatte) eingesteckt bzw. darin fixiert sind.

Auf diese Weise sind die einzelnen Werkzeug coaxial/parallelbeabstandet aneinandergereiht.

### Stand der Technik

Herkömmliche Werkzeugsets dieser Art für die Lagerung/Aufbewahrung von chirurgischen Werkzeugen gehören zum allgemeinen Stand der Technik. Diese bekannten Werkzeugsets weisen im Wesentlichen eine erste Lochplatte (ein sogenanntes Hülsenblech) auf, das als eine üblicherweise metallische Platte mit einer Lochmatrix aus runden Löchern unterschiedlicher Größe ausgebildet ist. In die Löcher sind Aufnahme-Expanderhülsen für die Aufnahme von Werkzeugen eingesteckt bzw. darin fixiert. Die Aufnahme-Expanderhülsen weisen jeweils eine Einstecköffnung für ein bestimmtes chirurgisches Werkzeug auf. Chirurgische Werkzeuge haben einen distalen Werkzeugeingriffsabschnitt und einen proximalen Schaftabschnitt, wobei Aufnahme-Expanderhülsen mit verschiedenen Durchmessern der Werkzeugaufnahmeöffnungen die korrekte und sitzgenaue Aufnahme von Werkzeugen mit verschiedenen Schaftdurchmessern erlauben. Zusätzlich kann das Werkzeugset eine mit der ersten Lochplatte verbundene zweite Lochplatte (ein sogenanntes Bodenblech) aufweisen, das parallel der ersten Lochplatte beabstandet ist und eine Einstecktiefe der chirurgischen Werkzeuge begrenzt.

Neben der schonenden Lagerung chirurgischer Werkzeuge ist die gründliche Reinigung und Sterilisation chirurgischer Werkzeuge von größter Wichtigkeit. Herkömmliche Aufnahme-Expanderhülsen sind als eine massive Kunststoffhülse mit elastisch radial-expandierbarem Inlay ausgebildet, wodurch das darin eingesteckte Werkzeug zwar sicher gehalten, jedoch eine ausreichende Umströmung der darin aufbewahrten Werkzeuge mit Reinigungsflüssigkeiten oder -gasen erschwert oder gar verhindert wird.

Die WO 2020/163481 A1 offenbart ein Caddy System zur Equipment Sterilisation. Das Caddy-System ist so konfiguriert und angepasst, dass es die Modularität fördert und der rauen Umgebung zentraler steriler Verarbeitungsprozesse standhält. Weiterhin kann es ohne zusätzliche Befestigungselemente oder Komponenten entfernt und auf dem Tablett neu positioniert werden.

Die DE 10 2004 053 355 A1 zeigt eine Wanne zur Aufnahme von sterilisierbedürftigen medizinischen Gegenständen. Die Wanne weist einen gelochten Boden auf, an welchem Fixiereinrichtungen angeordnet sind. Weiterhin kann an den Seitenwänden der Wanne ein gelochter Deckel angebracht werden, sodass Wasserstrahlen durch die Löcher der Wanne durchstrahlbar ist.

Die US 2013/0064709 A1 offenbart eine abnehmbare Tüllenvorrichtung und ein Verfahren zu dessen Verwendung. Eine Sterilisationsplattform weist eine Vielzahl von darin ausgebildeten Löchern auf. Eine Durchführungsvorrichtung/Tüllenvorrichtung ist in einem der mehreren Löcher angeordnet und dazu ausgelegt an ihrer Öffnung ein medizinisches Gerät aufzunehmen.

Die EP 0 263 011 B1 beschreibt einen Halter für zahnärztliche Schleif- und Fräswerkzeuge. Der Halter ist dazu ausgelegt an einem Träger befestigt zu werden und das in dem Halter eingesteckte Werkzeug einem Sterilisationsprozess zuzuführen.

Zur Verbesserung der Umströmung der Werkzeuge ist beispielsweise aus der EP 3 164 095 B1 eine Aufnahme-Expanderhülse bekannt, die einen mit radialen Durchbrüchen versehenen Hohlkörper aus Kunststoff sowie innerhalb des Hohlkörpers eingesetzte, nach innen ragende filigrane Klemmarme vorzugsweise aus Kunststoff oder Metall aufweist. Dabei dient der vergleichsweise massive Kunststoffhohlkörper zum Befestigen der Aufnahme-Expanderhülse an einer eine Lochplatte aufweisenden Haltungsvorrichtung, wohingegen die filigranen Klemmarme zum punktartigen Kontaktieren des in die Aufnahme-Expanderhülse eingesteckten Werkzeugs mit möglichst geringer Kontaktfläche dienen.

Zwar zeichnen sich diese bekannten Aufnahme-Expanderhülsen durch eine hohe Stabilität aus, haben jedoch auch weiterhin den Nachteil, dass der Kunststoffhohlkörper zwar radiale Durchbrüche aufweist, um das Eindringen und Umspülen des Reinigungsfluids zu ermöglichen, es aber immer noch möglich ist, dass sich beispielsweise Gewebereste und dergleichen Verunreinigungen an der Aufnahme-Expanderhülse sammeln können.

Ein weiterer entscheidender Nachteil an diesen bekannten Aufnahme-Expanderhülsen liegt darin, dass die Kunststoffhohlkörper in der Herstellung, Montage und Bestückung teuer und aufwändig sind. Um hohen Temperaturen bei der Sterilisation widerstehen zu können, müssen die Kunststoffteile aus speziellem temperaturresistentem Kunststoff gefertigt sein, was die Fertigungskosten erhöht. Um diese medizinische Anforderung erfüllen zu können, wird oftmals der Werkstoff PEEK verwendet, der als solcher kostenintensiv und schwer zu verarbeiten ist sowie eine begrenzte Lebensdauer aufweist. Zudem ist es bei einem mehrteiligen Aufbau der Aufnahme-Expanderhülse, insbesondere mit dem Kunststoffhohlkörper und den darin eingelassenen oder eingesetzten Metallklemmarmen, erforderlich, die einzelnen Bestandteile in manueller Handarbeit zusammenzuführen. Dies ist nicht nur zeitaufwändig, sondern birgt auch das Risiko der Beschädigung der Aufnahme-Expanderhülsen oder der fehlerhaften Zusammenführung bei der Montage. Zudem bleiben Verschmutzungen an Kunststoff aufgrund dessen positiver Oberflächenladung oft länger haften als an anderen Werkstoffen wie beispielsweise Metall, was die Reinigung der Aufnahme-Expanderhülse zusätzlich erschwert. Auch bei der Trocknung des Werkzeugsets nach der Reinigung erweist sich der massive Aufbau herkömmlicher Werkzeugsets und deren Fertigungsmaterial Kunststoff als nachteilig.

Die in den Kunststoffhohlkörper eingesetzten Metallklemmarme der aus dem Stand der Technik bekannten Werkzeugsets und Aufnahme-Expanderhülsen lassen sich jedoch ohne die Kunststoffhohlkörper nicht oder nicht stabil an der Halterungsvorrichtung der bekannten Werkzeugsets befestigen.

Es ist daher die Aufgabe der vorliegenden Offenbarung, eine Aufnahme-Expanderhülse eines Werkzeugsets sowie ein Werkzeugset bereitzustellen, welche einfach und kostengünstig herzustellen sind, eine effiziente Reinigung und/oder Sterilisation gewährleisten sowie eine einfach aufgebaute und gleichzeitig stabile Befestigung der Aufnahme-Expanderhülse an einer Halterungsvorrichtung des Werkzeugs ermöglichen.

Die Aufgabe der vorliegenden Offenbarung wird durch eine Aufnahme-Expanderhülse eines Werkzeugsets oder für ein Werkzeugset mit den Merkmalen des Patentanspruchs 1 sowie durch ein Werkzeugset mit den Merkmalen des nebengeordneten Patentanspruchs gelöst. Vorteilhafte Weiterbildungen sind Gegenstand der Unteransprüche.

Der Kern der Offenbarung besteht demzufolge darin, dass an der Aufnahme-Expanderhülse neben den Klemmarmen zum Haltern des Werkzeugs entweder an zusätzlichen (weiteren/separaten) Befestigungsarmen oder an jeweils einem Axialabschnitt der Klemmarme selbst Befestigungsabschnitte ausgebildet sind, um alleine daran die Aufnahme-Expanderhülse an der Halterungsvorrichtung zu befestigen. Diese Befestigungsabschnitte zeichnen sich insbesondere in der Weise aus, dass sie elastisch verformbar sowie radial gekrümmt oder (radial nach außen) abgewinkelt ausgebildet sind, so dass sie (aufgrund ihrer Elastizität) radial klemmend (und krallend) in die Halterungsvorrichtung eingesetzt werden können und in ihrem radial eingeklemmten Zustand durch ihre radiale Krümmung oder Abwinklung die Halterungsvorrichtung entgegen einer Einsteckrichtung des Werkzeugs hintergreifen können, so dass sie sowohl (radial) kraftschlüssig als auch (axial) formschlüssig an der Halterungsvorrichtung befestigbar sind.

Genauer gesagt dient die Aufnahme-Expanderhülse zur steckgelagerten, insbesondere senkrechten, Aufnahme chirurgischer Werkzeuge. Die Aufnahme-Expanderhülse (welche im Grunde genommen nur noch aus dem aus dem Stand der Technik per se bekannten Inlay besteht) weist eine Anzahl von (gleichmäßig) umfangsbeabstandeten, elastisch verformbaren Klemmarmen auf, die an einer offenen Stirnseite der Aufnahme-Expanderhülse eine Einstecköffnung für das Einstecken eines Werkzeugs ausbilden und (in jeweils einem Arm-Längsabschnitt) radial nach innen ragende Eingriffsabschnitte zur kraft- und/oder formschlüssigen Kontaktierung des Werkzeugs für das Halten des Werkzeugs haben. Die Klemmarme können vorzugsweise aus Metall gefertigt sein und in Umfangsrichtung der Aufnahme-Expanderhülse gleichverteilt angeordnet sein. Zudem weist die Aufnahme-Expanderhülse einen vorzugsweise metallischen Anschlag- oder Auffangboden auf, der an einem der Einstecköffnung gegenüberliegenden Endabschnitt der Aufnahme-Expanderhülse ausgebildet ist und von dem sich die Klemmarme aus in Axialrichtung zu der offenen Stirnseite hin erstrecken. Vorzugsweise können die Klemmarme an dem Auffangboden angeformt sein. Durch den Auffangboden kann eine Einstecktiefe des einzusteckenden Werkzeugs begrenzt werden.

Gemäß einem ggf. unabhängig zu beanspruchenden Aspekt der Offenbarung hat die Aufnahme-Expanderhülse zur form- und kraftschlüssigen Befestigung an einer eine Lochplatte aufweisenden Halterungsvorrichtung des Werkzeugsets eine Anzahl von Befestigungsabschnitten, die angepasst und vorgesehen sind, um unmittelbar mit der Lochplatte zum Befestigen an der Lochplatte zusammenzuwirken und die Lochplatte klemmend radial derart zu umgreifen, dass sie das Lochplattenmaterial in Lochplattendickenrichtung formschlüssig, beispielsweise entgegen einer Einsteckrichtung des einzusteckenden Werkzeugs, axial hintergreifen. Mit anderen Worten sind die Befestigungsabschnitte in Radialrichtung elastisch verformbar und radial abstehend oder gekrümmt ausgebildet, so dass die Befestigungsabschnitte in einem elastisch entspannten Zustand das Lochplattenmaterial in Lochplattendickenrichtung formschlüssig hintergreifen und in einem elastisch verformten Zustand zur Montage und Demontage der Aufnahme-Expanderhülse an der Halterungsvorrichtung an dem Lochplattenmaterial in Lochplattendickenrichtung vorbeiführbar sind (d.h. es nicht hintergreifen). Das heißt also, dass sich die Befestigungsabschnitte durch ihre Elastizität mit der Halterungsvorrichtung axial formschlüssig verhaken. Dies hat den Vorteil, dass die formschlüssige Befestigung durch die radiale elastische Klemmung lösbar und somit montierbar ist.

Gemäß einem zweiten Aspekt der Offenbarung, der auch gesondert und unabhängig von dem vorstehenden Aspekt beanspruchbar ist, kann die Aufnahme-Expanderhülse eine Anzahl von umfangsbeabstandeten, insbesondere elastisch verformbaren, vorzugsweise metallischen Befestigungsarmen, die sich von dem Auffangboden aus in Axialrichtung erstrecken und die an ihren freien, radial gebogenen Endabschnitten eine Anzahl von Befestigungsabschnitten oder die Befestigungsabschnitte ausbilden. Die Befestigungsabschnitte können demnach durch endseitige Befestigungsklauen an den Befestigungsarmen gebildet sein. Durch die Ausbildung der Befestigungsabschnitte an den freien Endabschnitten ist es einfach möglich, die Aufnahme-Expanderhülse, mit den Befestigungsabschnitten in einem radial elastisch verformten Zustand, axial in die Halterungsvorrichtung einzustecken, so dass die Befestigungsabschnitte nach dem axialen Einstecken der Aufnahme-Expanderhülse in einem radial entspannten Zustand (bzw. in einem der elastischen Verformung entgegenwirkenden Zustand) axial/ in Lochplattendickenrichtung (entgegen einer Einsteckrichtung des einzusteckenden Werkzeugs) die Halterungsvorrichtung formschlüssig hintergreifen.

Gemäß einer bevorzugten Ausführungsform des zweiten Aspekts können die Klemmarme und die Befestigungsarme in Umfangsrichtung der Aufnahme-Expanderhülse vorzugsweise regelmäßig abwechselnd angeordnet sein. Insbesondere können die Klemmarme und die Befestigungsarme jeweils gleichverteilt in Umfangsrichtung der Aufnahme-Expanderhülse angeordnet sein. Mit anderen Worten erstrecken sich die Befestigungsarme und die Klemmarme von dem Auffangboden aus sternförmig nach außen. Dies hat den Vorteil, dass die Aufnahme-Expanderhülse stabil an der Halterungsvorrichtung, beispielsweise zentriert in einem Loch der Halterungsvorrichtung, befestigt werden kann, und das einzusteckende Werkzeug senkrecht in der Aufnahme-Expanderhülse, beispielsweise zentriert dem durch die Klemmarme gebildeten Aufnahmeraum, gehalten werden kann.

Gemäß einer bevorzugten Ausführungsform des zweiten Aspekts können sich die Befestigungsarme vom Auffangboden aus in die gleiche Richtung wie die Klemmarme erstrecken oder in eine entgegengesetzte Richtung gegenläufig zu den Klemmarmen erstrecken. Bei einer gleichgerichteten Erstreckung der Befestigungsarme kann das Lochplattenmaterial insbesondere entgegen der Einsteckrichtung des Werkzeugs, d.h. von unten / aus Richtung des Auffangbodens, umgriffen werden. Bei einer entgegen gerichteten Erstreckung der Befestigungsarme kann das Lochplattenmaterial insbesondere in der Einsteckrichtung des Werkzeugs, d.h. von oben / aus Richtung der offenen Stirnseite, umgriffen werden.

Gemäß einer vorteilhaften Weiterbildung der bevorzugten Ausführungsform gemäß dem zweiten Aspekt können die Befestigungsabschnitte an radial nach außen gebogenen oder abstehenden Abschnitten der Befestigungsarme ausgebildet sein und dazu angepasst und vorgesehen sein, ein Loch der Lochplatte zu durchgreifen, so dass sie von radial innen elastisch klemmend das Lochplattenmaterial in Lochplattendickenrichtung hintergreifen. Das heißt, dass die Befestigungsabschnitte durch einen Krümmungsabschnitt gebildet sind, dessen Einstecköffnung radial nach außen hin ausgerichtet ist. Mit anderen Worten sind die Befestigungsabschnitte derart ausgebildet, dass sie zur Montage und Demontage an der Halterungsvorrichtung radial elastisch nach innen zusammengedrückt werden, so dass sie sich danach wieder radial aufspreizen und das Lochplattenmaterial hintergreifen.

Gemäß einer alternativen vorteilhaften Weiterbildung der bevorzugten Ausführungsform gemäß dem zweiten Aspekt können die Befestigungsabschnitte an radial nach innen gebogenen oder abstehenden Abschnitten der Befestigungsarme ausgebildet sein und dazu angepasst und vorgesehen sein, ein Loch der Lochplatte zu durchgreifen, so dass sie von radial außen klemmend das Lochplattenmaterial in Lochplattendickenrichtung hintergreifen. Das heißt, dass die Befestigungsabschnitte durch einen Krümmungsabschnitt gebildet sind, dessen Einstecköffnung radial nach innen hin ausgerichtet ist. Mit anderen Worten sind die Befestigungsabschnitte derart ausgebildet, dass sie zur Montage und Demontage an der Halterungsvorrichtung radial elastisch nach außen aufgespreizt werden, so dass sie sich danach wieder radial nach innen zusammenziehen und das Lochplattenmaterial hintergreifen.

Gemäß einem dritten Aspekt der Offenbarung, der auch gesondert und unabhängig von den vorstehenden Aspekten beanspruchbar ist, können die Klemmarme an einem ersten Axialabschnitt die Eingriffsabschnitte ausbilden und an einem sich in Axialrichtung unmittelbar daran anschließenden zweiten Axialabschnitt, der sich gegenüber dem ersten Axialabschnitt radial außerhalb befindet, eine Anzahl von Befestigungsabschnitten oder die Befestigungsabschnitte ausbilden. Das heißt, dass die Befestigungsabschnitte an den bereits vorhandenen, für das Halten des Werkzeugs erforderlichen Klemmarmen ausgebildet sein können. So kann die Aufnahme-Expanderhülse besonders kostengünstig hergestellt werden.

Gemäß einer bevorzugten Ausführungsform des dritten Aspekts kann der zweite Axialabschnitt an einem radial nach innen gebogenen, etwa U-förmigen Abschnitt, des Klemmarms ausgebildet sein und dazu angepasst und vorgesehen sein, ein Loch der Lochplatte zu durchgreifen, so dass er von radial innen klemmend das Lochplattenmaterial in Lochplattendickenrichtung hintergreift. Gemäß der bevorzugten Ausführungsform kann der U-förmige Abschnitt vorzugsweise eine nach radial außen ausgerichtete Öffnung besitzen. Mit anderen Worten sind die Befestigungsabschnitte derart ausgebildet, dass sie zur Montage und Demontage an der Halterungsvorrichtung radial elastisch nach innen zusammengedrückt werden, so dass sie sich danach wieder radial aufspreizen und das Lochplattenmaterial hintergreifen. Dadurch, dass der zweite Axialabschnitt von radial innen klemmend in die Lochplatte eingreift, wird der Klemmarm, an dem der zweite Axialabschnitt ausgebildet ist, insgesamt radial nach innen gedrückt. Dies hat den Vorteil, dass auch der Eingriffsabschnitt, der an dem ersten Axialabschnitt des Klemmarms ausgebildet ist, durch die Anbringung der Aufnahme-Expanderhülse nach radial innen gedrückt, wodurch sich wiederum die auf ein eingestecktes Werkzeug wirkende Haltekraft der Eingriffsabschnitte erhöht.

Gemäß einer bevorzugten Ausführungsform des dritten Aspekts kann der zweite Axialabschnitt an einem freien Endabschnitt des Klemmarms ausgebildet sein. Dies hat den Vorteil, dass sich die Eingriffsabschnitte zum Halten des Werkzeugs in Axialrichtung gesehen zwischen dem Auffangboden und den Befestigungsabschnitten befinden, so dass ein unbeabsichtigtes Verbiegen der Eingriffsabschnitte verhindert und damit das Werkzeug stabil gehalten werden kann. Zudem können die Befestigungsabschnitte so besonders einfach durch Biegen der Endabschnitte hergestellt werden. Ferner ist es durch die Ausbildung der Befestigungsabschnitte an den freien Endabschnitten einfach möglich, die Aufnahme-Expanderhülse, mit den Befestigungsabschnitten in einem radial elastisch verformten Zustand, axial in die Halterungsvorrichtung einzustecken, so dass die Befestigungsabschnitte nach dem axialen Einstecken der Aufnahme-Expanderhülse in einem radial entspannten Zustand (bzw. in einem der elastischen Verformung entgegenwirkenden Zustand) axial/ in Lochplattendickenrichtung (entgegen einer Einsteckrichtung des einzusteckenden Werkzeugs) die Halterungsvorrichtung formschlüssig hintergreifen. Gemäß einer alternativen bevorzugten Ausführungsform des dritten Aspekts kann der zweite Axialabschnitt zwischen dem Auffangboden und dem ersten Axialabschnitt des Klemmarms ausgebildet sein.

Gemäß einer Ausführungsform können die Klemmarme, die Befestigungsabschnitte und der Auffangboden einstückig aus einem biegeelastischen Metall, vorzugsweise einem Federstahl, beispielsweise aus dem Werkstoff 1.4310, ausgebildet sein. Durch die metallische Ausbildung kann die Aufnahme-Expanderhülse besonders effizient gereinigt und/oder sterilisiert werden sowie die Lebensdauer der Aufnahme-Expanderhülse verlängert werden.

Gemäß einer bevorzugten Ausführungsform kann die Aufnahme-Expanderhülse als ein, vorzugsweise lasergeschnittenes, Biegeumformteil ausgebildet sein. Dadurch kann die Aufnahme-Expanderhülse als Ganzes besonders einfach und kostengünstig vorzugsweise aus einem metallischen Werkstoff hergestellt werden. Durch die einstückige Ausbildung sind keine zusätzlichen Montageschritte zum Zusammenbau mehrerer Einzelteile notwendig.

Genauer gesagt dient das Werkzeugset zur steckgelagerten Aufnahme chirurgischer Werkzeuge. Das Werkzeugset weist die oben beschriebene Aufnahme-Expanderhülse sowie eine Halterungsvorrichtung auf, die eine erste Lochplatte mit ersten vorzugsweise runden Löchern zum Einstecken eines Werkzeugs sowie eine parallel dazu beabstandete mit der ersten Lochplatte verbundene zweite Lochplatte mit zweiten Löchern aufweist. Alternativ können die ersten Löcher auch rechteckig oder sternförmig ausgebildet sein. Vorzugsweise können manche oder alle der ersten Löcher und manche oder alle der zweiten Löcher zueinander fluchtend angeordnet sein. Die Aufnahme-Expanderhülse ist derart an der Haltevorrichtung form- und kraftschlüssig befestigt oder befestigbar, dass die Befestigungsabschnitte die erste Lochplatte und/oder die zweite Lochplatte radial klemmend derart umgreifen, dass sie das Lochplattenmaterial in Lochplattendickenrichtung formschlüssig, beispielsweise entgegen einer Einsteckrichtung des einzusteckenden Werkzeugs, axial hintergreifen. Mit anderen Worten sind die Befestigungsabschnitte in Radialrichtung elastisch verformbar und radial abstehend oder gekrümmt ausgebildet, so dass die Befestigungsabschnitte in einem elastisch entspannten Zustand das Lochplattenmaterial in Lochplattendickenrichtung formschlüssig hintergreifen und in einem elastisch verformten Zustand zur Montage und Demontage der Aufnahme-Expanderhülse an der Halterungsvorrichtung an dem Lochplattenmaterial in Lochplattendickenrichtung vorbeiführbar sind (d.h. es nicht hintergreifen). Das heißt also, dass sich die Befestigungsabschnitte durch ihre Elastizität mit der Halterungsvorrichtung axial formschlüssig verhaken. Dies hat den Vorteil, dass die formschlüssige Befestigung durch die radiale elastische Klemmung lösbar und somit montierbar ist.

Gemäß einer bevorzugten Ausführungsform kann zumindest eines der ersten Löcher und/oder der zweiten Löcher, in das die Aufnahme-Expanderhülse eingesteckt oder einsteckbar ist, radial nach innen ragende Trennstege haben, die eine formschlüssige Verdrehsicherung für die Befestigungsabschnitte bilden. So wird ein unbeabsichtigtes Verdrehen der Aufnahme-Expanderhülse gegenüber der Halterungsvorrichtung ausgeschlossen.

Gemäß einer Weiterbildung der bevorzugten Ausführungsform hat das Loch, in das die Aufnahme-Expanderhülse eingesteckt oder einsteckbar ist, je Befestigungsabschnitt zwei Trennstege, die jeweils einen Anschlag in einer Umfangsrichtung der Aufnahme-Expanderhülse bilden. Das heißt, dass jeder der Befestigungsabschnitte in Umfangsrichtung der Aufnahme-Expanderhülse zwischen zwei Trennstege angeordnet ist. Dadurch kann die Befestigung der Aufnahme-Expanderhülse an der Halterungsvorrichtung und somit auch die Lage des einzusteckenden Werkzeugs stabilisiert werden.

Gemäß einer bevorzugten Weiterbildung der bevorzugten Ausführungsform können die Trennstege derart ausgebildet sein, insbesondere (etwa jeweils zwei Trennstege) derart miteinander verbunden sein, dass sie, insbesondere zusammen mit dem Außendurchmesser des Lochs, mehrere im Wesentlichen dreieckige oder kreissektorförmige Befestigungsabschnittsperforationen bilden, durch die jeweils ein Befestigungsabschnitt (axial, vorzugsweise entgegen der Einsteckrichtung) durchführbar ist, und/oder eine sternförmige Klemmarmperforation bilden, die vorzugsweise sich von der Lochmitte sternförmig radial nach außen erstreckende Ausweichspalte hat, deren Breite größer als die Klemmarmbreite ist, so dass die Klemmarme innerhalb der Klemmarmperforation, vorzugsweise durch die Trennstege geführt, elastisch in Radialrichtung verlagerbar sind. Dies hat den Vorteil, dass die Lage des einzusteckenden Werkzeugs stabilisiert und ein unbeabsichtigtes beispielsweise plastisches Verformen der Klemmarme, insbesondere quer zu der Radialrichtung, verhindert werden kann. Zudem können die Trennstege einen Innendurchmesser bilden, der einen Schaftdurchmesser des einzusteckenden Werkzeugs begrenzt, so dass ein Neigen des Werkzeugs in der Aufnahme-Expanderhülse verhindert oder eingeschränkt werden kann.

Gemäß einer alternativen bevorzugten Weiterbildung der bevorzugten Ausführungsform können die Trennstege derart ausgebildet sein, insbesondere derart miteinander verbunden sind, dass sie sich an der Lochmitte überschneiden und sternförmig von der Lochmitte radial nach außen erstrecken und dadurch, insbesondere zusammen mit dem Außendurchmesser des Lochs, mehrere im Wesentlichen dreieckige oder kreissektorförmige Lochabschnitte bilden. Dies hat den Vorteil, dass an der Lochmitte Material der Lochplatte vorhanden ist, auf welchem der Auffangboden aufliegen kann und das von den Befestigungsabschnitten, die Lochabschnitte durchgreifend, radial von außen klemmend umgriffen werden kann.

Gemäß einer bevorzugten Ausführungsform können die ersten Löcher der ersten Lochplatte unterschiedliche Durchmesser aufweisen, die einen Schaftdurchmesser des einzusteckenden Werkzeugs begrenzen. Die ersten Löcher können vorzugsweise rund oder sternförmig sein. So kann ein Neigen des Werkzeugs in der Aufnahme-Expanderhülse verhindert oder eingeschränkt werden.

Mit anderen Worten betrifft die Offenbarung eine Steckhülse für ein Sandwichblech, bei der eine ein einzusteckendes Werkzeug federnd lagernde Metallklammer mittels mehreren, beispielsweise drei Armen an einer ersten Lochplatte (mit ersten Löchern zum Einstecken des Werkzeugs) oder einer (beabstandet mit der ersten Lochplatte verbundenen) zweiten Lochplatte einer Halterungsvorrichtung durch Klemmen und Hintergreifen fixiert wird und (in Einsteckrichtung des Werkzeugs) in die zweite Lochplatte eintaucht. Zusätzlich kann die Metallklammer mittels Punktschweißen oder Kleben an der ersten Lochplatte und/oder der zweiten Lochplatte befestigt werden. Eine Form der ersten Löcher kann die Lage des Werkzeugs stabilisieren, insbesondere das Neigen des Werkzeugs verhindern, und/oder durch unterschiedliche Innendurchmesser die Werkzeugschaftdurchmesser eingrenzen. Eine Form der zweiten Löcher bzw. ein Abstand der zweiten Lochplatte kann eine Einstecktiefe der Werkzeuge eingrenzen. Vorzugsweise kann ein Verdrehen der Metallklammer durch die Form der ersten Lochplatte und/oder der zweiten Lochplatte verhindert sein.

### Kurzbeschreibung der Figuren

Fign. 1 bis 23 zeigen verschiedene Ansichten und Modifikationen einer form- und kraftschlüssig befestigbaren Aufnahme-Expanderhülse sowie eines Werkzeugsets mit der Aufnahme-Expanderhülse gemäß einem Aspekt der Offenbarung.
Fign. 24 bis 26 zeigen verschiedene Ansichten und Modifikationen einer formschlüssig einhängbaren Aufnahme-Expanderhülse sowie eines Werkzeugsets mit der Aufnahme-Expanderhülse gemäß einem anderen Aspekt der Offenbarung.
Fign. 27 bis 38 zeigen verschiedene Ansichten und Modifikationen einer stoffschlüssig befestigbaren Aufnahme-Expanderhülse sowie eines Werkzeugsets mit der Aufnahme-Expanderhülse gemäß einem weiteren Aspekt der Offenbarung.

### Beschreibung der Ausführungsformen

Nachstehend werden Ausführungsformen der vorliegenden Offenbarung auf der Basis der zugehörigen Figuren beschrieben.

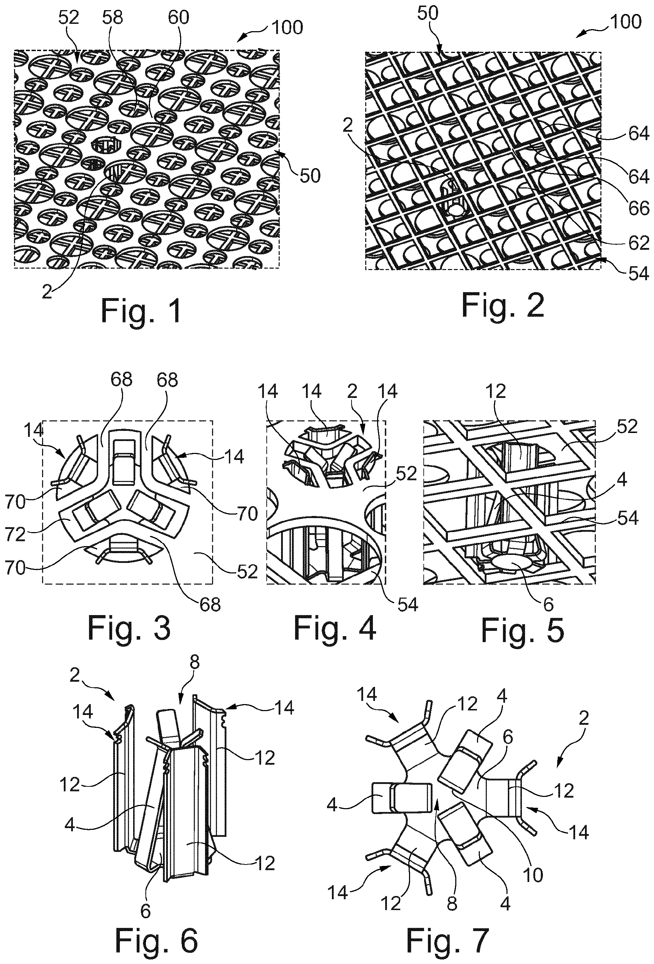

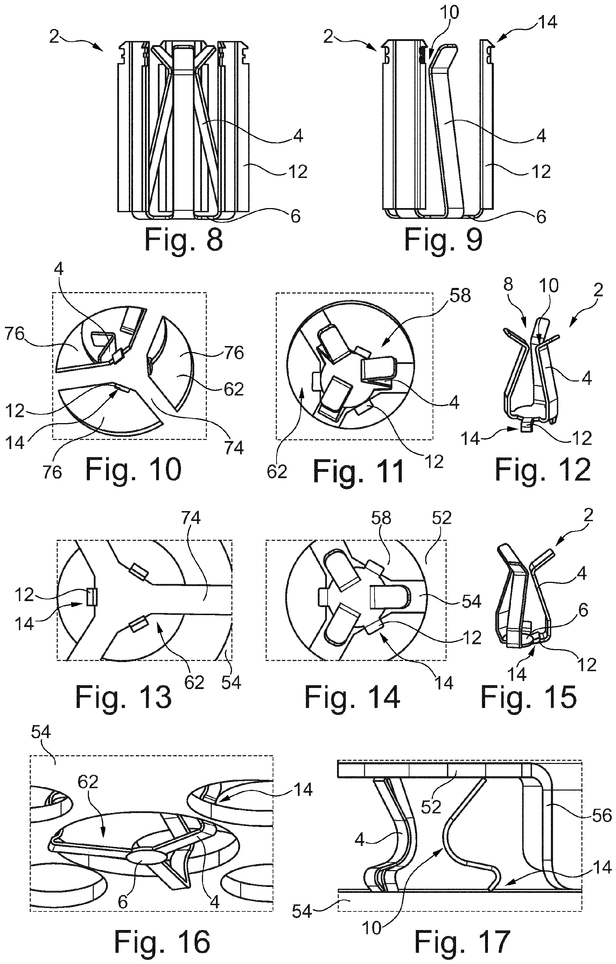

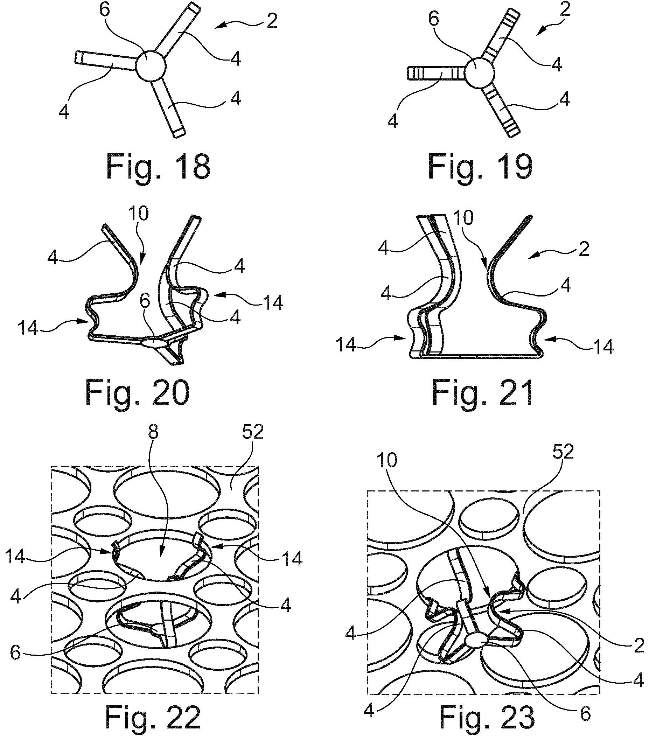

Fign. 1 bis 23 zeigen eine erste Ausführungsform der vorliegenden Offenbarung in verschiedenen Modifikationen. Fign. 1 bis 9 zeigen eine erste Modifikation der ersten Ausführungsform. Fign. 10 bis 12 zeigen eine zweite Modifikation der ersten Ausführungsform. Fign. 13 bis 15 zeigen eine dritte Modifikation der ersten Ausführungsform. Fing. 16 bis 21 zeigen eine vierte Modifikation der ersten Ausführungsform. Fign. 22 und 23 zeigen eine fünfte Modifikation der ersten Ausführungsform.

Fign. 1 bis 5 zeigen eine Aufnahme-Expanderhülse 2 eines Werkzeugsets 100, die in eine Halterungsvorrichtung 50 des Werkzeugsets 100 eingesetzt bzw. eingesteckt ist. Fign. 6 bis 9 zeigen eine Darstellung der Aufnahme-Expanderhülse 2, die nicht in der Halterungsvorrichtung 50 des Werkzeugsets 100 eingesteckt bzw. eingesetzt ist.

Die Aufnahme-Expanderhülse 2 hat eine Anzahl von umfangsbeabstandeten Klemmarmen 4 und einen vorzugsweise ebenen Auffangboden 6, von dem sich die Klemmarme 4 aus in Axialrichtung, d.h. senkrecht zu dem Auffangboden 6, erstrecken. Alternativ kann der Auffangboden 6 auch gewölbt sein oder eine Prägung, einen Überstand oder dergleichen aufweisen, auch wenn dies nicht dargestellt ist. In der dargestellten Ausführungsform hat die Aufnahme-Expanderhülse 2 drei Klemmarme 4. Alternativ könnte die Aufnahme-Expanderhülse 2 auch mehr als 3, beispielsweise 4, 5 oder 6 Klemmarme 4 haben. Die Klemmarme 4 sind elastisch verformbar und bilden an einer offenen Stirnseite der Aufnahme-Expanderhülse 2 eine Einstecköffnung 8 für das Einstecken eines (nicht dargestellten) Werkzeugs aus. Die Klemmarme 4 können gleichverteilt über den Umfang der Aufnahme-Expanderhülse 2 angeordnet sein. Vorzugsweise sind die Klemmarme 4 biegeelastisch und aus einem Metall, beispielsweise aus einem Federstahl, wie dem Werkstoff 1.4310, gefertigt. Die Klemmarme 4 bilden an ihren (stirnseitigen) freien Endabschnitten vorzugsweise einen Trichter, der sich in Einsteckrichtung des aufzunehmenden Werkzeugs verengt.

Die Klemmarme 4 haben radial nach innen ragende Eingriffsabschnitte 10 zur kraft- und/oder formschlüssigen Kontaktierung des Werkzeugs, um das Werkzeug zu halten. Durch Einstecken des Werkzeugs (und das Kontaktieren der Eingriffsabschnitt 10) werden die Klemmarme 4 in Radialrichtung der Aufnahme-Expanderhülse 2 federelastisch nach außen gedrückt. Durch die dadurch resultierende elastische Spannung der Klemmarme 4 wird das eingesteckte Werkzeug zwischen den Klemmarmen 4 gehalten/festgeklemmt. Das heißt, dass ein Außendurchmesser eines radial innerhalb der Aufnahme-Expanderhülse 2 gebildeten Aufnahmeraums zur Aufnahme des Werkzeugs durch die Eingriffsabschnitte 10 bzw. der elastischen Verformbarkeit der Klemmarme 4 nach radial außen begrenzt ist. Hierbei sind die Eingriffsabschnitte 10 vorzugsweise so ausgebildet, dass die Kontaktfläche zwischen den Klemmarmen 4 und dem aufgenommenen Werkzeug möglichst gering (linien- und/oder punktförmig) ist. Da die Oberfläche des Werkzeugs nur an den durch die Eingriffsabschnitte 10 definierten Punkten/Linien eine Kontaktstelle mit der Aufnahme-Expanderhülse 2 bildet, ist das Werkzeug großflächig von Reinigungsfluid umspülbar.

Die Aufnahme-Expanderhülse 2 hat den Auffangboden 6, der an dem der Einstecköffnung 8 gegenüberliegenden Endabschnitt der Aufnahme-Expanderhülse 2 ausgebildet ist. Der Auffangboden 6 erstreckt sich im Wesentlichen plattenförmig senkrecht zur Axialrichtung der Aufnahme-Expanderhülse 2. Alternativ kann der Auffangboden 6 auch mit einer Prägung ausgeführt sein. In der dargestellten Ausführungsform hat der Auffangboden 6 die Form eines Kreises. Alternativ könnte der Auffangboden 6 beispielsweise ringförmig, oval oder im Wesentlichen dreieckig ausgebildet sein. Der Auffangboden 6 könnte auch mit einem oder mehreren Löchern versehen sein oder mit einer zentralen, im Wesentlichen kegelstumpfförmigen Vertiefung, in die ein Ende eines Werkzeugs selbstzentrierend aufgenommen werden kann. Die Kegelstumpffläche dieser Vertiefung kann dabei auch geschlitzt bzw. unterbrochen ausgeführt sein und die "Spitze" des Kegelstumpfes kann offen oder geschlossen ausgebildet sein. Die Klemmarme 4 erstrecken sich von der radialen Außenkante des Auffangbodens 6 aus in Axialrichtung zu der offenen Stirnseite der Aufnahme-Expanderhülse 2 hin. Vorzugsweise ist der Auffangboden 6 aus einem Metall, beispielsweise aus einem Federstahl, wie dem Werkstoff 1.4310, gefertigt. Beispielsweise können die Klemmarme 4 über einen spitzwinkligen Eckverbinder/einen spitzwinkligen Eckverbindungsbereich/eine Biegestelle an dem Auffangboden 6 angebracht sein, so dass der Neigungswinkel an dem Eckverbinder zwischen den Klemmarmen 4 und dem Auffangboden 6 durch das Einstecken des Werkzeugs entgegen der Elastizität der Klemmarme 4 vergrößert wird. Insbesondere kann der Auffangboden 6 integral/werkstoffeinstückig mit den Klemmarmen 4 verbunden sein.

Die Aufnahme-Expanderhülse 2 kann in die Halterungsvorrichtung 50 des Werkzeugsets 100 eingesteckt werden. Insbesondere kann die Aufnahme-Expanderhülse 2 unmittelbar an der Halterungsvorrichtung 50 befestigt werden. Die Halterungsvorrichtung 50 weist eine erste Lochplatte (Lochmatrix/Hülsenblech/Siebstruktur) 52 und eine zweite Lochplatte (Gitterplatte/Lochmatrix/Bodenblech/Siebstruktur) 54 auf. Die beiden Lochplatten 52, 54 sind zueinander parallel beabstandet angeordnet und miteinander beispielsweise über Außenwände (einen Rahmen) 56 (vgl. Fig. 17 oder Fign. 27 und 28) oder Stege verbunden.

Die erste Lochplatte 52 hat eine Anzahl erster Löcher (Aussparungen/Perforationen) 58. Die ersten Löcher 58 sind in der dargestellten Ausführungsform im Wesentlichen rund ausgebildet und können zur Aufnahme unterschiedlicher Werkzeugschaftdurchmesser unterschiedliche Durchmesser haben. Alternativ können die ersten Löcher 58 auch eine im Wesentlichen rechteckige oder quadratische Form haben oder sternförmig sein, auch wenn dies nicht dargestellt ist. Die ersten Löcher 58 sind durch Material der ersten Lochplatte 52, beispielsweise durch erste Stege 60, voneinander getrennt. Beispielsweise können die ersten Löcher 58, wie in Fig. 1 dargestellt, in geraden Reihen angeordnet sein. Das heißt, dass die Lochmitten der ersten Löcher 58 benachbarter Reihen und/oder die Lochmitten von benachbarten ersten Löchern 58 einer Reihe jeweils den gleichen Abstand haben, d.h. die gleiche Lochteilung besitzen. In Fig. 1 ist die Aufnahme-Expanderhülse 2 in eines der ersten Löcher 58 in Einsteckrichtung des Werkzeugs eingesetzt, so dass es in Einsteckrichtung des Werkzeugs, d.h. in Richtung zu der zweiten Lochplatte 54 hin, in die erste Lochplatte 52 eintaucht.

Die zweite Lochplatte 54 hat eine Anzahl zweiter Löcher (Aussparungen/Perforationen) 62. Die zweiten Löcher 62 sind im Wesentlichen quadratisch (vgl. Fig. 2) oder im Wesentlichen rund ausgebildet (vgl. Fign. 10, 13 und 16). Die zweiten Löcher 62 sind durch Material der zweiten Lochplatte 54, beispielsweise durch zweite Stege 64, voneinander getrennt. Vorzugsweise bei quadratischer Form können die zweiten Löcher 62 beispielsweise in geraden Reihen angeordnet sein. Das heißt, dass die Lochmitten der zweiten Löcher 62 benachbarter Reihen und/oder die Lochmitten von benachbarten zweiten Löchern 62 einer Reihe jeweils den gleichen Abstand haben, d.h. die gleiche Lochteilung besitzen. Das heißt, dass die zweiten Stege 64 geradlinig sowie parallel bzw. senkrecht zueinander angeordnet sind. Die zweiten Stege 64 schneiden sich an ihren Kreuzungspunkten 66.

Die ersten Löcher 58 und die zweiten Löcher 62 können in Axialrichtung zueinander fluchtend angeordnet sein. Alternativ können die zweiten Löcher 62 auch in Axialrichtung nicht fluchtend zu den ersten Löchern 58 angeordnet sein, wobei vorzugsweise bei nicht fluchtender Anordnung die Kreuzungspunkte 66 in Axialrichtung fluchtend zu den ersten Löchern 58 angeordnet sind.

Zumindest eines der ersten Löcher 58 kann in einer vorteilhaften Weiterbildung (vgl. Fign. 3 und 4) radial nach innen ragende, durch Material der ersten Lochplatte 52 gebildete Trennstege 68 aufweisen. Die Trennstege 68 dienen als formschlüssige Verdrehsicherung für die Aufnahme-Expanderhülse 2. Vorzugsweise weist das zumindest eine Loch der ersten Löcher 58 (je später detailliert beschriebenem Befestigungsabschnitt 14) zumindest zwei radial nach innen ragende Trennstege 68 auf, die jeweils einen Anschlag in einer Umfangsrichtung der Aufnahme-Expanderhülse 2 bilden. Das heißt, dass jeder der Befestigungsabschnitte 14 in Umfangsrichtung der Aufnahme-Expanderhülse 2 zwischen zwei Trennstegen 68 angeordnet ist. Durch die Trennstege 68 wird das zumindest eine Loch der ersten Löcher 58 in mehrere voneinander getrennte Lochabschnitte unterteilt. Vorzugsweise können die Trennstege 68 derart ausgebildet sein, insbesondere (etwa jeweils zwei Trennstege 68) derart miteinander verbunden sein, dass sie, insbesondere zusammen mit dem Außendurchmesser des Lochs, mehrere im Wesentlichen dreieckige oder kreissektorförmige Befestigungsabschnittsperforationen 70 bilden, durch die jeweils ein Befestigungsabschnitt 14 (axial, vorzugsweise entgegen der Einsteckrichtung) durchführbar ist. Zudem oder alternativ können die Trennstege 68 derart ausgebildet sein, insbesondere (etwa jeweils zwei Trennstege 68) derart miteinander verbunden sein, dass sie, insbesondere zusammen mit dem Außendurchmesser des Lochs, eine sternförmige Klemmarmperforation 72 bilden. Die Klemmarmperforation 72 hat vorzugsweise sich von der Lochmitte sternförmig radial nach außen erstreckende Ausweichspalte, deren Breite größer als eine Breite der Klemmarme 4 ist. Dadurch können die Klemmarme 4 innerhalb der Klemmarmperforation 72, vorzugsweise durch die Trennstege 68 geführt, elastisch in Radialrichtung verlagert werden. Vorzugsweise hat die Klemmarmperforation 72 zumindest so viele Ausweichspalte wie die Aufnahme-Expanderhülse 2 Klemmarme 4 hat.

Die zweiten Löcher 62 können in einer vorteilhaften Weiterbildung (vgl. Fign. 10 und 11 oder Fign. 13 und 14) radial nach innen ragende, durch Material der zweiten Lochplatte 54 gebildete Trennstege 74 aufweisen. Vorzugsweise weist zumindest ein Loch der zweiten Löcher 62 die radial nach innen ragenden Trennstege 74 auf, die das unterteilte zweite Loch 62 in mehrere Lochabschnitte 76 unterteilen. Die Trennstege 74 können sich vorzugsweise in einem Mittelpunkt des unterteilten zweiten Lochs 62 schneiden. Die Lochabschnitte 76 weisen vorzugsweise die gleiche Form auf und können beispielsweise im Wesentlichen dreieckig oder kreissektorförmig ausgebildet sein. Das heißt, dass die Trennstege 74 derart ausgebildet sein können, insbesondere derart miteinander verbunden sein können, dass sie sich an der Lochmitte überschneiden und sternförmig von der Lochmitte radial nach außen erstrecken und dadurch, insbesondere zusammen mit dem Außendurchmesser des Lochs, die mehreren im Wesentlichen dreieckigen oder kreissektorförmigen Lochabschnitte 76 bilden.

Die Aufnahme-Expanderhülse 2 hat mehrere, beispielsweise an Befestigungsarmen 12 (vgl. Fign. 1 bis 15) ausgebildete Befestigungsabschnitte 14, die zur kraft- und formschlüssigen Befestigung der Aufnahme-Expanderhülse 2 an der Halterungsvorrichtung 50 des Werkzeugsets dienen. In der dargestellten Ausführungsform hat die Aufnahme-Expanderhülse 2 drei umfangsbeabstandete Befestigungsarme 12. Alternativ könnte die Aufnahme-Expanderhülse 2 auch mehr als drei Befestigungsarme 12 haben. Die Befestigungsarme 12 können gleichverteilt über den Umfang der Aufnahme-Expanderhülse 2 angeordnet sein. Vorzugsweise entspricht die Anzahl der Befestigungsarme 12 der Anzahl der Klemmarme 4. Insbesondere können die Befestigungsarme 12 in Umfangsrichtung der Aufnahme-Expanderhülse 2 abwechselnd mit den Klemmarmen 4 angeordnet sein. Vorzugsweise sind die Befestigungsarme 12 aus einem Metall, beispielsweise aus einem Federstahl, wie dem Werkstoff 1.4310, gefertigt. Beispielsweise können die die Befestigungsarme 12 fest, insbesondere über einen Eckverbinder/einen Eckverbindungsbereich/eine Biegestelle an dem Auffangboden 6 angebracht sein. Insbesondere können die Befestigungsarme 12 integral/werkstoffeinstückig mit dem Auffangboden 6 und/oder mit den Klemmarmen 4 verbunden sein.

Die Befestigungsabschnitte 14 dienen insbesondere zur unmittelbaren form- und kraftschlüssigen Befestigung an der Halterungsvorrichtung 50 des Werkzeugsets 100, insbesondere an der ersten Lochplatte 52 und/oder der zweiten Lochplatte 54. Die Befestigungsabschnitte 14 sind angepasst und vorgesehen, um unmittelbar mit der ersten Lochplatte 52 oder zweiten Lochplatte 54 zum Befestigen an dieser Lochplatte 52 zusammenzuwirken und diese Lochplatte 52, 54 radial klemmend derart zu umgreifen, dass sie das Lochplattenmaterial in Lochplattendickenrichtung formschlüssig, etwa entgegen einer Einsteckrichtung des einzusteckenden Werkzeugs, axial hintergreifen.

Der Auffangboden 6 der Aufnahme-Expanderhülse 2 ist durch eine ebene Platte gebildet. Alternativ kann der Auffangboden 6 auch gewölbt sein, beispielsweise im Wesentlichen kegelstumpfförmig sein, oder eine Prägung, einen Überstand oder dergleichen aufweisen, auch wenn dies nicht dargestellt ist. Eine Unterseite 16 des Auffangbodens 6 ist eine von den Klemmarmen 4 abgewandte Axialseite des Auffangsbodens 6, d.h. eine axiale Außenfläche der Aufnahme-Expanderhülse 2. Eine Oberseite 18 des Auffangbodens 6 ist eine den Klemmarmen 4 zugewandte Axialseite des Auffangsbodens 6, d.h. eine (zwischen den Klemmarmen 4 angeordnete) axiale Innenfläche der Aufnahme-Expanderhülse 2. Die Oberseite 18 des Auffangbodens 6 bildete eine axiale Anschlagsfläche für das einzusteckende Werkzeug.

In der in Fign. 1 bis 9 dargestellten ersten Modifikation erstrecken sich die Befestigungsarme 12 von dem Auffangboden 6 aus in Axialrichtung und bilden an ihren freien, radial nach außen gebogenen Endabschnitten die Befestigungsabschnitte 14, etwa in Form von nach radial außen ausgerichteten Rastnasen, aus. Dabei erstrecken sich die Befestigungsarme 12 vom Auffangboden 6 aus in die gleiche Richtung wie die Klemmarme 4. Die in Fig. 1 bis 9 dargestellten Befestigungsarme 12 erstrecken sich im Wesentlichen genauso weit in Axialrichtung wie die Klemmarme 4. Insbesondere entspricht eine Axialerstreckung der in Fig. 1 bis 9 dargestellten Befestigungsarme 12 im Wesentlichen einem Abstand zwischen der ersten Lochplatte 52 und der zweiten Lochplatte 54. Die radial nach außen gebogenen oder abstehenden Endabschnitte, welche die Befestigungsabschnitte 14 ausbilden, sind dazu angepasst und vorgesehen, eines der ersten Löcher 58 zu durchgreifen, so dass sie von radial innen klemmend das Lochplattenmaterial der ersten Lochplatte 52 in Lochplattendickenrichtung hintergreifen. Mit anderen Worten umklammern die Befestigungsabschnitte 14 die erste Lochplatte 52 von unten.

In der in Fign. 10 bis 12 dargestellten zweiten Modifikation erstrecken sich die Befestigungsarme 12 von dem Auffangboden 6 aus in Axialrichtung erstrecken und bilden an ihren freien, radial nach innen gebogenen Endabschnitten die Befestigungsabschnitte 14, etwa in Form von Befestigungsklauen, aus. Dabei erstrecken sich die Befestigungsarme 12 vom Auffangboden 6 aus in eine entgegengesetzte Richtung gegenläufig zu den Klemmarmen 4. Die in Fig. 10 bis 12 dargestellten Befestigungsarme 12 sind in Axialrichtung (wesentlich) kürzer als die Klemmarme 4 ausgebildet. Insbesondere entspricht eine Axialerstreckung der in Fig. 10 bis 12 dargestellten Befestigungsarme 12 im Wesentlichen der Dicke der zweiten Lochplatte 54. Die radial nach innen gebogenen oder abstehenden Endabschnitte, welche die Befestigungsabschnitte 14 ausbilden, sind dazu angepasst und vorgesehen, eines der zweiten Löcher 62 (bzw. deren Lochabschnitte 74) zu durchgreifen, so dass sie von radial außen klemmend das Lochplattenmaterial der zweiten Lochplatte 54 in Lochplattendickenrichtung hintergreifen. Der Auffangboden 6 liegt mit seiner Unterseite 16 axial auf Lochplattenmaterial der zweiten Lochplatte 54 auf. Mit anderen Worten umklammern die Befestigungsabschnitte 14 die zweite Lochplatte 54 von oben.

In der in Fign. 13 bis 15 dargestellten dritten Modifikation erstrecken sich die Befestigungsarme 12 von dem Auffangboden 6 aus in Axialrichtung erstrecken und bilden an ihren freien, radial nach innen gebogenen Endabschnitten die Befestigungsabschnitte 14, etwa in Form von Befestigungsklauen, aus. Dabei erstrecken sich die Befestigungsarme 12 vom Auffangboden 6 aus in die gleiche Richtung wie die Klemmarme 4. Die in Fig. 13 bis 15 dargestellten Befestigungsarme 12 sind in Axialrichtung (wesentlich) kürzer als die Klemmarme 4 ausgebildet. Insbesondere entspricht eine Axialerstreckung der in Fig. 13 bis 15 dargestellten Befestigungsarme 12 im Wesentlichen der Dicke der zweiten Lochplatte 54. Die radial nach innen gebogenen oder abstehenden Endabschnitte, welche die Befestigungsabschnitte 14 ausbilden, sind dazu angepasst und vorgesehen, eines der zweiten Löcher 62 (bzw. deren Lochabschnitte 74) zu durchgreifen, so dass sie von radial außen klemmend das Lochplattenmaterial der zweiten Lochplatte 54 in Lochplattendickenrichtung hintergreifen. Der Auffangboden 6 liegt mit seiner Oberseite 16 axial an dem Lochplattenmaterial der zweiten Lochplatte 54 an. Mit anderen Worten umklammern die Befestigungsabschnitte 14 die zweite Lochplatte 54 von unten.

In der in Fign. 16 bis 21 dargestellten vierten Modifikation bilden die Klemmarme 4 an einem ersten Axialabschnitt die Eingriffsabschnitte 10 aus und an einem sich in Axialrichtung unmittelbar daran anschließenden zweiten Axialabschnitt die Befestigungsabschnitte 14, etwa in Form von Krümmungsabschnitten, aus. Der zweite Axialabschnitt befindet sich gegenüber dem ersten Axialabschnitt radial außerhalb. Der zweite Axialabschnitt ist zwischen dem Auffangboden und dem ersten Axialabschnitt des Klemmarms 4 ausgebildet. Der zweite Axialabschnitt ist ein radial nach innen gebogener, etwa U-förmiger Abschnitt, des Klemmarms 4 und dazu angepasst und vorgesehen, eines der zweiten Löcher 62 der zweiten Lochplatte 54 zu durchgreifen, so dass er von radial innen klemmend das Lochplattenmaterial in Lochplattendickenrichtung hintergreift bzw. umgreift. Der U-förmige Abschnitt kann vorzugsweise eine nach radial außen ausgerichtete Öffnung besitzen. Im montierten Zustand befindet sich der Auffangboden 6 der Aufnahme-Expanderhülse 2 vorzugsweise unterhalb der zweiten Lochplatte 54 (d.h. außerhalb des Zwischenraums zwischen der ersten Lochplatte 52 und der zweiten Lochplatte 54).

In der in Fign. 22 und 23 dargestellten fünften Modifikation bilden die Klemmarme 4 an einem ersten Axialabschnitt die Eingriffsabschnitte 10 aus und an einem sich in Axialrichtung unmittelbar daran anschließenden zweiten Axialabschnitt die Befestigungsabschnitte 14 aus. Der zweite Axialabschnitt befindet sich gegenüber dem ersten Axialabschnitt radial außerhalb. Der zweite Axialabschnitt ist an einem freien Endabschnitt des Klemmarms 4 ausgebildet. Der zweite Axialabschnitt ist ein radial nach innen gebogener, etwa U-förmigen Abschnitt, des Klemmarms 4 ist und dazu angepasst und vorgesehen, eines der ersten Löcher 58 der ersten Lochplatte 52 zu durchgreifen, so dass er von radial innen klemmend das Lochplattenmaterial in Lochplattendickenrichtung hintergreift bzw. umgreift. Der U-förmige Abschnitt kann vorzugsweise eine nach radial außen ausgerichtete Öffnung besitzen. Die in Fign. 22 bis 23 dargestellte fünfte Modifikation der Aufnahme-Expanderhülse 2 kann an einer als Einzelblech/Einzellochplatte ausgebildeten Halterungsvorrichtung 50 befestigt werden.

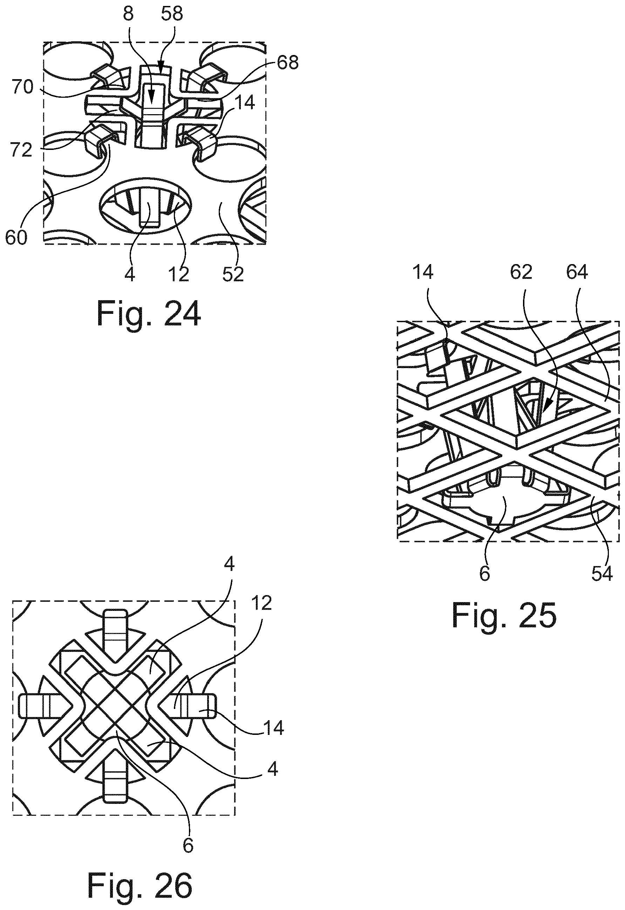

Fign. 24 bis 26 zeigen eine zweite Ausführungsform der vorliegenden Offenbarung. Die in die Haltevorrichtung 50 eingehängte Aufnahme-Expanderhülse 2 der zweiten Ausführungsform ist in Fig. 24 in einer perspektivischen Ansicht von oben, in Fig. 25 in einer perspektivischen Ansicht von unten und in Fig. 26 in einer Draufsicht dargestellt.

Die Aufnahme-Expanderhülse 2 hat mehrere, in der dargestellten Ausführungsform vier, umfangsbeabstandete Klemmarme 4. Alternativ könnte die Aufnahme-Expanderhülse 2 beispielsweise auch drei Klemmarme 4 oder mehr als vier Klemmarme 4 haben. Die Klemmarme 4 können gleichverteilt über den Umfang der Aufnahme-Expanderhülse 2 angeordnet sein. Die Klemmarme 4 sind elastisch verformbar und bilden an der offenen Stirnseite der Aufnahme-Expanderhülse 2 die Einstecköffnung 8 für das Einstecken des (nicht dargestellten) Werkzeugs aus. Vorzugsweise sind die Klemmarme 4 biegeelastisch und aus einem Metall, beispielsweise aus einem Federstahl, wie dem Werkstoff 1.4310, gefertigt. Die Klemmarme 4 bilden an ihren (stirnseitigen) freien Endabschnitten vorzugsweise einen Trichter, der sich in Einsteckrichtung des aufzunehmenden Werkzeugs verengt.

Die Klemmarme 4 haben die radial nach innen ragende Eingriffsabschnitte 10 zur kraft- und/oder formschlüssigen Kontaktierung des Werkzeugs, um das Werkzeug zu halten. Durch Einstecken des Werkzeugs (und das Kontaktieren des Eingriffsabschnitts 10) werden die Klemmarme 4 in Radialrichtung federelastisch nach außen gedrückt. Durch die dadurch resultierende elastische Spannung der Klemmarme 4 wird das eingesteckte Werkzeug zwischen den Klemmarmen 4 gehalten/festgeklemmt. Das heißt, dass ein Außendurchmesser eines radial innerhalb der Aufnahme-Expanderhülse 2 gebildeten Aufnahmeraums zur Aufnahme des Werkzeugs durch die Eingriffsabschnitte 10 bzw. der elastischen Verformbarkeit der Klemmarme 4 nach radial außen begrenzt ist. Hierbei sind die Eingriffsabschnitte 10 vorzugsweise so ausgebildet, dass die Kontaktfläche zwischen den Klemmarmen 4 und dem aufgenommenen Werkzeug möglichst gering (linien- und/oder punktförmig) ist. Da die Oberfläche des Werkzeugs nur an den durch die Eingriffsabschnitte 10 definierten Punkten/Linien eine Kontaktstelle mit der Aufnahme-Expanderhülse 2 bildet, ist das Werkzeug großflächig von Reinigungsfluid umspülbar.

Die Aufnahme-Expanderhülse 2 hat den Auffangboden 6, der an dem der Einstecköffnung 8 gegenüberliegenden Endabschnitt der Aufnahme-Expanderhülse 2 ausgebildet ist. Der Auffangboden 6 kann als Axialanschlag dienen und ein zu tiefes Einstecken des Werkzeugs verhindern. Der Auffangboden 6 erstreckt sich im Wesentlichen plattenförmig senkrecht zur Axialrichtung der Aufnahme-Expanderhülse 2. In der dargestellten Ausführungsform hat der Auffangboden 6 die Form eines Kreises. Alternativ könnte der Auffangboden 6 beispielsweise ringförmig, oval oder im Wesentlichen dreieckig ausgebildet sein. Der Auffangboden 6 kann eben oder beispielsweise im Wesentlichen kegelstumpfförmig ausgebildet sein. Von dem Auffangboden 6 aus erstrecken sich die Klemmarme 4 in Axialrichtung zu der Einstecköffnung 8 hin. Die Klemmarme 4 erstrecken sich von der radialen Außenkante des Auffangbodens 6 aus. Vorzugsweise ist der Auffangboden 6 aus einem Metall, beispielsweise aus einem Federstahl, wie dem Werkstoff 1.4310, gefertigt. Beispielsweise können die Klemmarme 4 über einen spitzwinkligen Eckverbinder/einen spitzwinkligen Eckverbindungsbereich/eine Biegestelle an dem Auffangboden 6 angebracht sein, so dass der Neigungswinkel an dem Eckverbinder zwischen den Klemmarmen 4 und dem Auffangboden 6 durch das Einstecken des Werkzeugs entgegen der Elastizität der Klemmarme 4 vergrößert wird. Insbesondere kann der Auffangboden 6 integral/werkstoffeinstückig mit den Klemmarmen 4 verbunden sein.

Die Aufnahme-Expanderhülse 2 hat mehrere Befestigungsarme 12, die zur Befestigung der Aufnahme-Expanderhülse 2 an der Halterungsvorrichtung 50 des Werkzeugsets dienen. In der dargestellten Ausführungsform hat die Aufnahme-Expanderhülse 2 vier umfangsbeabstandete Befestigungsarme 12. Alternativ könnte die Aufnahme-Expanderhülse 2 beispielsweise auch drei Befestigungsarme 12 oder mehr als vier Befestigungsarme 12 haben. Die Befestigungsarme 12 können gleichverteilt über den Umfang der Aufnahme-Expanderhülse 2 angeordnet sein. Vorzugsweise entspricht die Anzahl der Befestigungsarme 12 der Anzahl der Klemmarme 4. Insbesondere können die Befestigungsarme 12 in Umfangsrichtung abwechselnd mit den Klemmarmen 4 angeordnet sein. Vorzugsweise sind die Befestigungsarme 12 aus einem Metall, beispielsweise aus einem Federstahl, wie dem Werkstoff 1.4310, gefertigt. Beispielsweise können die die Befestigungsarme 12 fest, insbesondere über einen Eckverbinder/einen Eckverbindungsbereich/eine Biegestelle an dem Auffangboden 6 angebracht sein. Insbesondere können die Befestigungsarme 12 integral/werkstoffeinstückig mit dem Auffangboden 6 und/oder mit den Klemmarmen 4 verbunden sein. Die Befestigungsarme 12 erstrecken sich von dem Auffangboden 6 aus in Axialrichtung zu der Einstecköffnung 8 hin. Die Befestigungsarme 12 erstrecken sich von der radialen Außenkante des Auffangbodens 6 aus. Vorzugsweise ist die Aufnahme-Expanderhülse 2 als ein metallisches Biegeumformteil ausgebildet.

Die Befestigungsarme 12 haben an ihren an der offenen Stirnseite der Aufnahme-Expanderhülse 2 angeordneten Endabschnitten jeweils einen hakenartigen Befestigungsabschnitt 14 zur einhängenden Befestigung an der Halterungsvorrichtung 50. Der Befestigungsabschnitt 14 ist derart gebogen oder abgewinkelt ausgebildet, dass er (in Einsteckrichtung des Werkzeugs) an der Halterungsvorrichtung 50 einhängbar ist. Vorzugsweise kann der Befestigungsabschnitt 14 derart ausgebildet und vorgesehen sein, dass er die Halterungsvorrichtung 50 radial und axial hintergreift. Mit anderen Worten umgreift der Befestigungsabschnitt 14 die Halterungsvorrichtung 50 axial und radial, insbesondere zur Festlegung der Radialposition und der Axialposition der Aufnahme-Expanderhülse 2 an der Halterungsvorrichtung 50.

Insbesondere kann der Befestigungsabschnitt 14 ein U-förmiges Profil bilden. In der dargestellten Ausführungsform erstreckt sich ein Basisabschnitt, der einen Grund des U-förmigen Profils bildet, im Wesentlichen in Radialrichtung (d.h. senkrecht zu der Axialrichtung). Der Basisabschnitt liegt also flächig an der Halterungsvorrichtung 50 an. Schenkel des U-förmigen Profils erstrecken sich von den Enden des Basisabschnitts aus im Wesentlichen in Axialrichtung in die gleiche Richtung. Ein radial innerer Schenkel der beiden Schenkel kann sich in Axialrichtung länger, vorzugsweise bis zu dem Auffangboden 6 hin, erstrecken als ein radial äußerer Schenkel der beiden Schenkel. Das heißt, dass das Profil der Befestigungsabschnitte 14 eine Einstecköffnung (Einhängeöffnung) hat, die vorzugsweise radial nach außen und/oder vorzugsweise axial zu dem Auffangboden hin, d.h. entgegen der Einsteckrichtung des Werkzeugs, ausgerichtet sein kann. Durch die radial nach außen ausgerichtete Einstecköffnung des U-Profils können die Befestigungsabschnitte 14 von radial innen an der Halterungsvorrichtung 50 angebracht werden. Durch die axial entgegen der Einsteckrichtung des Werkzeugs ausgerichtete Einstecköffnung des U-Profils können die Befestigungsabschnitte 14 in der Einsteckrichtung des Werkzeugs an der Halterungsvorrichtung 50 angebracht werden.

Der Auffangboden 6 der Aufnahme-Expanderhülse 2 ist beispielsweise durch eine ebene Platte gebildet. Alternativ kann der Auffangboden 6 auch gewölbt sein, beispielsweise im Wesentlichen kegelstumpfförmig sein, oder eine Prägung, einen Überstand oder dergleichen aufweisen, auch wenn dies nicht dargestellt ist. Die Unterseite 16 des Auffangbodens 6 ist die von den Klemmarmen 4 abgewandte Axialseite des Auffangsbodens 6, d.h. eine axiale Außenfläche der Aufnahme-Expanderhülse 2. Die Oberseite 18 des Auffangbodens 6 ist die den Klemmarmen 4 zugewandte Axialseite des Auffangsbodens 6, d.h. eine (zwischen den Klemmarmen 4 angeordnete) axiale Innenfläche der Aufnahme-Expanderhülse 2. Die Oberseite 18 des Auffangbodens 6 bildet eine axiale Anschlagsfläche für das einzusteckende Werkzeug.

Die Halterungsvorrichtung 50 der zweiten Ausführungsform weist die erste Lochplatte 52 auf. Die erste Lochplatte 52 hat eine Anzahl erster Löcher (Aussparungen/Perforationen) 58, die in der dargestellten Ausführungsform rund ausgebildet sind und zur Aufnahme unterschiedlicher Werkzeugschaftdurchmesser unterschiedliche Durchmesser haben. Alternativ können die ersten Löcher 58 auch eine im Wesentlichen rechteckige oder quadratische Form haben oder sternförmig sein, auch wenn dies nicht dargestellt ist. Die ersten Löcher 58 sind durch erste Stege 60 voneinander getrennt. Beispielsweise können die ersten Löcher 58, wie in Fig. 24 dargestellt, in 60° versetzten Reihen angeordnet sein. Das heißt, dass die Lochmitten benachbarter Reihen mittig zueinander versetzt angeordnet sind. Die Halterungsvorrichtung 50 kann die zweite Lochplatte 54 aufweisen, die parallel zu der ersten Lochplatte 52 beabstandet angeordnet ist und mit der ersten Lochplatte 52, beispielsweise über den in Fign. 24 bis 26 nicht dargestellten Rahmen, verbunden ist. Die zweite Lochplatte 54 hat eine Anzahl zweiter Löcher (Aussparungen/Perforationen) 62, die eine quadratische Form haben können. Beispielsweise können die Löcher 62, wie in Fig. 25 dargestellt, in geraden Reihen angeordnet sein. Die Löcher 62 können von geradlinigen Stegen 64 voneinander getrennt sein. Die zweite Lochplatte 54 kann als Verdrehsicherung für die Aufnahme-Expanderhülse 2 dienen. Die in Fign. 24 bis 26 dargestellte zweite Ausführungsform der Aufnahme-Expanderhülse 2 kann auch an einer als Einzelblech/Einzellochplatte ausgebildeten/nur die erste Lochplatte 52 aufweisenden Halterungsvorrichtung 50 befestigt werden.

Die Aufnahme-Expanderhülse 2 der zweiten Ausführungsform ist an der ersten Lochplatte 52 der Halterungsvorrichtung 50 befestigt. Insbesondere ist die Aufnahme-Expanderhülse 2 in eines der ersten Löcher 58 eingesteckt. Der hakenartige Befestigungsabschnitt 14 hintergreift den Steg 60 axial und radial, so dass er, insbesondere der radial äußere Schenkel des U-förmigen Profils, in ein benachbartes erstes Loch 58 formschlüssig eingreift. Mit anderen Worten durchgreift der Befestigungsabschnitt 14 das erste Loch 58, in das die Aufnahme-Expanderhülse 2 eingesteckt ist, radial nach außen abgewinkelt, so dass er sich an dem Lochplattenmaterial parallel/entlang nach außen erstreckt und den das erste Loch 58 begrenzenden Steg 60 axial hintergreift, und ist axial in Richtung zu dem Auffangboden 6 hin abgewinkelt, so dass er in das benachbarte erste Loch 58 formschlüssig eingreift und den das erste Loch 58 begrenzenden Steg 60 radial hintergreift. Somit umgreift der Befestigungsabschnitt 14 das Lochplattenmaterial in Axialrichtung, insbesondere entgegen der Einsteckrichtung des Werkzeugs. Das heißt, dass die Befestigungsabschnitte 14 an den Stegen 60 der ersten Lochplatte 52 eingehängt sind.

Zumindest eines der ersten Löcher 58 kann in einer vorteilhaften Weiterbildung die radial nach innen ragenden, durch Material der ersten Lochplatte 52 gebildeten Trennstege 68 aufweisen. Die Trennstege 68 bilden eine formschlüssige Verdrehsicherung für die Aufnahme-Expanderhülse 2, insbesondere für die Befestigungsabschnitte 14. Vorzugsweise weist das zumindest eine Loch der ersten Löcher 58 (je Befestigungsabschnitt 14) zumindest zwei radial nach innen ragende Trennstege 68 auf, die jeweils einen Anschlag in einer Umfangsrichtung der Aufnahme-Expanderhülse 2 bilden. Das heißt, dass jeder der Befestigungsabschnitte 14 in Umfangsrichtung der Aufnahme-Expanderhülse 2 zwischen zwei Trennstegen 68 angeordnet ist.

Durch die Trennstege 68 wird das zumindest eine Loch der ersten Löcher 58 in mehrere voneinander getrennte Lochabschnitte unterteilt. Vorzugsweise können die Trennstege 68 derart ausgebildet sind, insbesondere (etwa jeweils zwei Trennstege 68) derart miteinander verbunden sein, dass sie, insbesondere zusammen mit dem Außendurchmesser des Lochs, mehrere im Wesentlichen dreieckige oder kreissektorförmige Befestigungsabschnittsperforationen 70 bilden. Jede Befestigungsabschnittsperforation 70 ist auf den Befestigungsabschnitt 14, insbesondere der Basisabschnitt des U-Profils, derart abgestimmt, dass der Befestigungsabschnitt 14 durch die Befestigungsabschnittsperforation 70 axial (vorzugsweise entgegen der Einsteckrichtung) durchführbar ist. Zudem oder alternativ können die Trennstege 68 derart ausgebildet sein, insbesondere (etwa jeweils zwei Trennstege 68) derart miteinander verbunden sein, dass sie, insbesondere zusammen mit dem Außendurchmesser des Lochs, eine sternförmige Klemmarmperforation 72 bilden. Die Klemmarmperforation 72 hat vorzugsweise sich von der Lochmitte sternförmig radial nach außen erstreckende Ausweichspalte, deren Breite größer als eine Breite der Klemmarme 4 ist. Dadurch können die Klemmarme 4 innerhalb der Klemmarmperforation 72, vorzugsweise durch die Trennstege 68 geführt, elastisch in Radialrichtung (begrenzt) verlagert werden. Vorzugsweise hat die Klemmarmperforation 72 zumindest so viele Ausweichspalte wie die Aufnahme-Expanderhülse 2 Klemmarme 4 hat. In der in Fign. 24 bis 26 dargestellten Ausführungsform mit vier Klemmarmen 4 hat die Klemmarmperforation 72 einen plusförmigen/kreuzförmigen Querschnitt. Ein durch die Trennstege 68 gebildeter Innendurchmesser kann den Schaftdurchmesser des einzusteckenden Werkzeugs begrenzen.

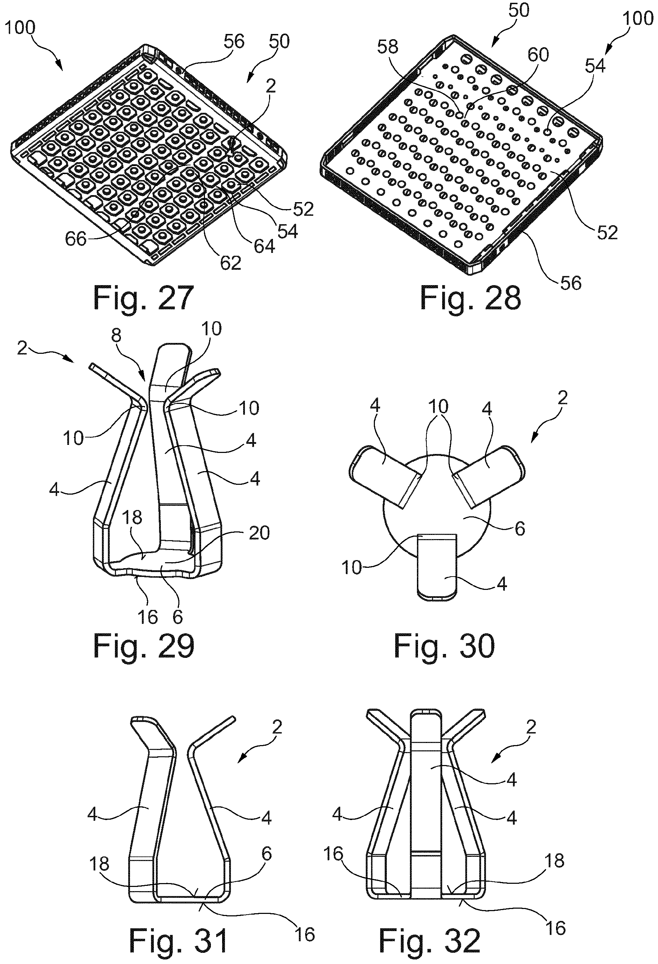

In Fign. 27 bis 38 ist eine dritte Ausführungsform dargestellt. Die Halterungsvorrichtung 50 der dritten Ausführungsform ist in Fig. 27 perspektivisch von unten und in Fig. 28 perspektivisch von oben dargestellt. Die erste Lochplatte (Lochmatrix/Hülsenblech/Siebstruktur) 52 und die zweite Lochplatte (Gitterplatte/Lochmatrix/Bodenblech/Siebstruktur) 54 sind zueinander parallel beabstandet angeordnet und miteinander über die Außenwände (Rahmen) 56 verbunden.

Die erste Lochplatte 52 hat eine Anzahl erster Löcher (Aussparungen/Perforationen) 58, die in der dargestellten Ausführungsform rund ausgebildet sind und zur Aufnahme unterschiedlicher Werkzeugschaftdurchmesser unterschiedliche Durchmesser haben. Alternativ können die ersten Löcher 58 auch eine im Wesentlichen rechteckige oder quadratische Form haben oder sternförmig sein, auch wenn dies nicht dargestellt ist. Alternativ könnten die ersten Löcher 58 mit gleichem Durchmesser ausgebildet sein. In jedes der ersten Löcher 58 ist jeweils eine Aufnahme-Expanderhülse 2 einsteckbar/einsetzbar bzw. eingesteckt/eingesetzt. Die ersten Löcher 58 sind durch erste Stege 60 voneinander getrennt.

Die zweite Lochplatte 54 hat eine Anzahl zweiter Löcher (Aussparungen/Perforationen) 62, die im Wesentlichen viereckig, vorzugsweise im Wesentlichen rechteckig oder quadratisch, ausgebildet sind und voneinander durch die zweiten Stege 64 getrennt werden. In der dargestellten Ausführungsform hat jedes der zweite Löcher 62 hat dieselbe Form. Alternativ könnten die zweiten Löcher 62 unterschiedliche Größen aufweisen.

Die zweiten Stege 62 erstrecken sich geradlinig und parallel zu den Außenkanten der zweiten Lochplatte 54. Die zweiten Stege 62 bilden eine Gitterstruktur und schneiden sich im Wesentlichen rechtwinklig an ihren Kreuzungspunkten 66. Die zweiten Löcher 62 sind in Reihen angeordnet und die zweiten Löcher 62 direkt benachbarter Reihen sind zueinander, vorzugsweise mittig, versetzt angeordnet. Somit sind die zweiten Löcher 62 in 60°-versetzten Reihen angeordnet. Das heißt, dass die zweiten Stege 64 an ihren Kreuzungspunkten 66 eine T-Kreuzung bilden bzw. dass jeder Kreuzungspunkt 66 den Rand von drei aneinander angrenzenden zweiten Löchern 62, insbesondere zwei Eckpunkte und eine Seitenkante, bildet. An den Kreuzungspunkten 66 verbreitern sich die zweiten Stege 64 an den Eckpunkten der zweiten Löcher 62 konvex nach außen, d.h. dass sie sich konvex in Richtung zur Lochmitte hin wölben. Dadurch ergibt sich, dass die zweiten Löcher 62 eine im Wesentlichen quadratische Form haben, deren Ecken konkav nach innen gewölbt sind (deren Eckpunkte sich etwa viertelkreisförmig nach innen wölben). Anders gesagt sind die zweiten Stege 64 derart verbreitert ausgebildet, dass an ihren Kreuzungspunkten 66 eine Kreisfläche, deren Radius im Wesentlichen der Breite der zweiten Stege 64 entspricht, vollständig zwischen den zweiten Löchern 62 angeordnet werden kann. Mit anderen Worten bilden die zweiten Stege 64 an den Kreuzungspunkten 66 eine Schweiß-, Löt- oder Klebfläche, die vorzugsweise im Wesentlichen so groß wie eine Fläche des Auffangbodens 6 ist.

Die ersten Löcher 58 sind zu den Kreuzungspunkten 66 der zweiten Lochplatte 54, insbesondere jeweils zu dem Mittelpunkt der zwischen den zweiten Löchern 62 ausgebildeten Kreisflächen, fluchtend angeordnet. Das heißt, dass die ersten Löcher 58 zu den zweiten Löchern 60 versetzt (d.h. nicht fluchtend) angeordnet sind. Insbesondere können die ersten Löcher 58 der ersten Lochplatte 52 in parallel beabstandeten Reihen angeordnet sein und die Lochmitten jeweils zwei benachbarter Reihen um eine Stegbreite der zweiten Stege 64 zueinander versetzt angeordnet sein.

Fign. 29 bis 32 zeigen verschiedene Ansichten der Aufnahme-Expanderhülse 2 der dritten Ausführungsform. Die Aufnahme-Expanderhülse 2 weist die elastisch verformbaren Klemmarme 4 auf. Die Klemmarme 4 bilden an ihren (stirnseitigen) freien Endabschnitten vorzugsweise einen Trichter, der sich in Einsteckrichtung des aufzunehmenden Werkzeugs verengt. Durch Einstecken des Werkzeugs wird der Durchmesser des durch die Klemmarme 4 radial begrenzten Hohlraums radial aufgeweitet und bilden Anlaufschrägflächen für das einzusteckende Werkzeug und die Klemmarme 4 werden in Radialrichtung federelastisch nach außen gedrückt. Durch die dadurch resultierende elastische Spannung der Klemmarme 4 wird das eingesteckte Werkzeug in dem Hohlraum zwischen den Klemmarmen 4 festgeklemmt. Hierbei sind die Klemmarme 4 vorzugsweise so ausgebildet, dass die Kontaktfläche zwischen den Klemmarmen und dem aufgenommenen Werkzeug möglichst gering (linien- und/oder punktförmig) ist. Da die Oberfläche des Werkzeugs nur an den durch die Eingriffsabschnitte 10 definierten Punkten/Linien eine Kontaktstelle mit der Aufnahme-Expanderhülse 2 bildet, ist das Werkzeug großflächig von Reinigungsfluid umspülbar.

Die Aufnahme-Expanderhülse 2 hat den Auffangboden 6, der an dem der Einstecköffnung 8 gegenüberliegenden Endabschnitt der Aufnahme-Expanderhülse 2 ausgebildet ist. Der Auffangboden 6 kann als Axialanschlag dienen und ein zu tiefes Einstecken des Werkzeugs verhindern. Der Auffangboden 6 erstreckt sich im Wesentlichen plattenförmig senkrecht zur Axialrichtung der Aufnahme-Expanderhülse 2. In der dargestellten Ausführungsform hat der Auffangboden 6 die Form eines Kreises. Alternativ könnte der Auffangboden 6 beispielsweise ringförmig, oval oder im Wesentlichen dreieckig ausgebildet sein. Von dem Auffangboden 6 aus erstrecken sich die Klemmarme 4 in Axialrichtung zu der offenen Stirnseite hin. Die Klemmarme 4 erstrecken sich von der radialen Außenkante des Auffangbodens 6 aus in Axialrichtung. Vorzugsweise ist der Auffangboden 6 aus einem Metall, beispielsweise aus einem Federstahl, wie dem Werkstoff 1.4310, gefertigt. Beispielsweise können die Klemmarme 4 über einen spitzwinkligen Eckverbinder/einen spitzwinkligen Eckverbindungsbereich/eine Biegestelle an dem Auffangboden 6 angebracht sein, so dass der Neigungswinkel an dem Eckverbinder zwischen den Klemmarmen 4 und dem Auffangboden 6 durch das Einstecken des Werkzeugs entgegen der Elastizität der Klemmarme 4 vergrößert wird. Insbesondere kann der Auffangboden 6 integral/werkstoffeinstückig mit den Klemmarmen 4 verbunden sein.

Der Auffangboden 6 ist bei einer unmittelbaren stoffschlüssigen Befestigung der Aufnahme-Expanderhülse 2 an der Halterungsvorrichtung 50 durch eine ebene, abkantfreie Platte gebildet, von der sich ausschließlich die Klemmarme 4 axial, d.h. in einer Richtung senkrecht zu der Platte, wegerstrecken und die in einem Mittenbereich an ihrer einen oder beiden Flachseiten einen Anschweiß-, Löt- oder Klebabschnitt 20 ausbildet. Mit anderen Worten besteht die Aufnahme-Expanderhülse 2 aus dem ebenen, abkantfreien Auffangboden 6, von dem sich die mehreren, in der dargestellten Ausführungsform drei, Klemmarme 4 axial in Richtung zu der offenen Stirnseite hin erstrecken. Somit erstrecken sich von der radialen Außenkante des Auffangbodens 6 ausschließlich die Klemmarme 4 in Axialrichtung.

Die Unterseite 16 des Auffangbodens 6 ist die von den Klemmarmen 4 abgewandte Axialseite des Auffangsbodens 6, d.h. eine axiale Außenfläche der Aufnahme-Expanderhülse 2. Die Oberseite 18 des Auffangbodens 6 ist die den Klemmarmen 4 zugewandte Axialseite des Auffangsbodens 6, d.h. eine (zwischen den Klemmarmen 4 angeordnete) axiale Innenfläche der Aufnahme-Expanderhülse 2. Die Oberseite 18 des Auffangbodens 6 bildet eine axiale Anschlagsfläche für das eingesteckte Werkzeug. Die Unterseite 16 und/oder die Oberseite 18 des Auffangbodens 6 besitzen oder bilden den vorzugsweise metallischen Anschweiß-, Löt- oder Klebabschnitt 20 aus, der zum unmittelbaren stoffschlüssigen, insbesondere (punkt-)schweißenden, Befestigen der Aufnahme-Expanderhülse 2 an der Halterungsvorrichtung 50, insbesondere der zweiten Lochplatte 54, angepasst und vorgesehen ist. Bei einer Ausbildung des Anschweiß-, Löt- oder Klebabschnitts 20 an der Unterseite 16 kann die Aufnahme-Expanderhülse 2 axial von oben (in der Einsteckrichtung des einzusteckenden Werkzeugs) auf die Halterungsvorrichtung 50 aufgesetzt/aufgelegt und stoffschlüssig daran befestigt werden. Bei einer Ausbildung des Anschweiß-, Löt- oder Klebabschnitts 20 an der Oberseite 18 kann die Aufnahme-Expanderhülse 2 axial von unten (entgegen der Einsteckrichtung des einzusteckenden Werkzeugs) auf die Halterungsvorrichtung 50 aufgesteckt und stoffschlüssig daran befestigt werden. Mit anderen Worten ist die Unterseite 16 und/oder die Oberseite 18 des Auffangbodens 6 an der Halterungsvorrichtung 50 angeschweißt oder anschweißbar.

Die Aufnahme-Expanderhülse 2 kann derart angepasst und vorgesehen sein, dass sie auf die zweite Lochplatte 54 der Halterungsvorrichtung 50 entgegen einer Einsteckrichtung des einzusteckenden Werkzeugs aufsteckbar ist, so dass die Klemmarme 4 durch die zweiten Löcher 62 hindurchgreifen und der Auffangboden 6 axial von der zweiten Lochplatte 54 als Abstandshalter hervorsteht. Mit anderen Worten kann die zweite Lochplatte 54 dadurch beispielsweise gegenüber einem (nicht dargestellten) Aufnahmekasten, in den die zweite Lochplatte 54 in der Einsteckrichtung des Werkzeugs eingesetzt wird, beabstandet gehalten werden. Das heißt, dass eine Unterseite der zweiten Lochplatte 54 um die Dicke des Auffangbodens 6 von dem Aufnahmekasten axial beabstandet gehalten werden kann. Gemäß einer Ausführungsform kann der Auffangboden 6 einen schweißbaren Einsatz oder eine schweißbare Beschichtung aufweisen.

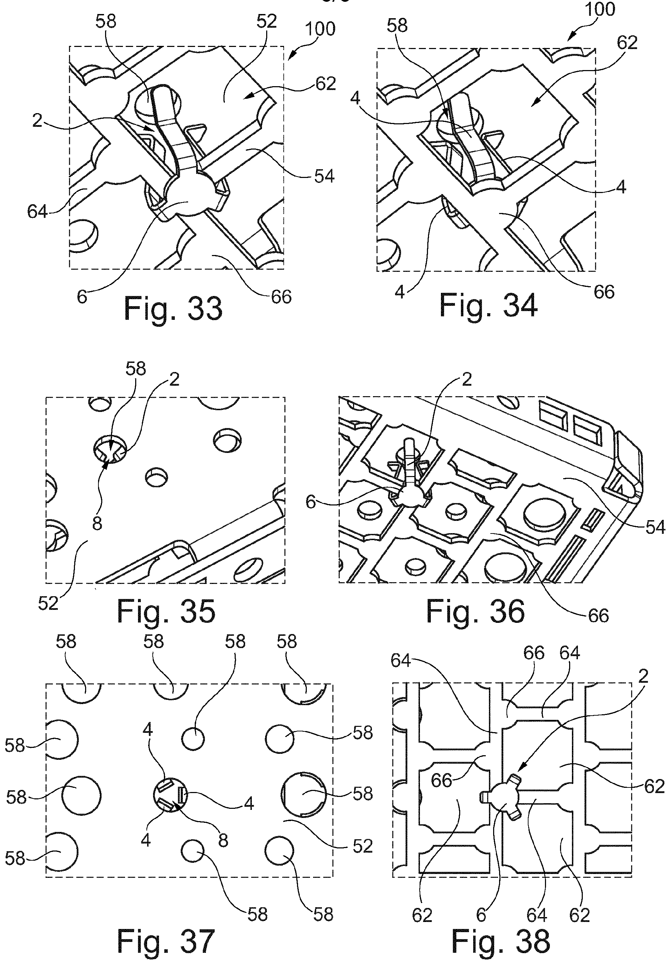

Fign. 33 bis 38 zeigen verschiedene Ansichten des Werkzeugsets 100 der dritten Ausführungsform, bei dem die Aufnahme-Expanderhülse 2 an der Halterungsvorrichtung 50 befestigt ist. In einer in Fign. 33, 36 und 38 gezeigten Weiterbildung der dritten Ausführungsform ist die Oberseite 18 des Auffangbodens 6 an der Halterungsvorrichtung 50, insbesondere an einem der Kreuzungspunkte 66 der zweiten Lochplatte 54 befestigt, vorzugsweise (durch Punktschweißen) angeschweißt. In einer in Fig. 34 gezeigten Weiterbildung der dritten Ausführungsform ist die Unterseite 16 des Auffangbodens 6 an der Halterungsvorrichtung 50, insbesondere an einem der Kreuzungspunkte 66 der zweiten Lochplatte 54 befestigt, vorzugsweise (durch Punktschweißen) angeschweißt.

In der in Fig. 34 gezeigten Weiterbildung ist die Aufnahme-Expanderhülse 2 (von oben, d.h. in Einsteckrichtung des Werkzeugs) auf die zweite Lochplatte 54 aufgesetzt. Das heißt, dass die Aufnahme-Expanderhülse 2 vollständig zwischen den beiden Lochplatten 52, 54 angeordnet sein kann. Der Auffangboden 6 und die durch den Kreuzungspunkt 66 gebildete Kreisfläche sind zentriert zueinander ausgerichtet. Die Unterseite/axiale Außenfläche 16 der Aufnahme-Expanderhülse 2 ist an der der ersten Lochplatte 52 zugewandte Axialseite der zweiten Lochplatte 54 angebracht, insbesondere durch Punktschweißen.

In der in Fign. 33, 36 und 38 gezeigten Weiterbildung ist die Aufnahme-Expanderhülse 2 (von unten, d.h. entgegen der Einsteckrichtung des Werkzeugs) auf die zweite Lochplatte 54 aufgesteckt bzw. in die zweite Lochplatte 54 eingesteckt. Der Auffangboden 6 und die durch den Kreuzungspunkt 66 gebildete Kreisfläche sind zentriert zueinander ausgerichtet. Die Oberseite/axiale Innenfläche 18 der Aufnahme-Expanderhülse 2 ist an der der ersten Lochplatte 52 abgewandten Axialseite der zweiten Lochplatte 54 angebracht, insbesondere durch Punktschweißen. Mit anderen Worten durchgreift jeder der Klemmarme 4 eines der zweiten Löcher 62 axial. Das heißt, dass die Aufnahme-Expanderhülse 2 axial an der der ersten Lochplatte 52 abgewandten Axialseite der zweiten Lochplatte 54 um die Dicke des Auffangbodens 6 hervorsteht. Dies hat den Vorteil, dass der Auffangboden 6 als ein Abstandshalter zwischen der zweiten Lochplatte 54 und dem (nicht dargestellten) Aufnahmekasten dienen kann.

Durch die fluchtende Anordnung der ersten Löcher 58 mit den Kreuzungspunkten 66 der zweiten Lochplatte fluchtet die durch die Klemmarme 4 gebildete Einstecköffnung 8 mit den ersten Löchern 58. So kann das einzusteckende Werkzeug in der Einsteckrichtung durch die erste Lochplatte 52 axial hindurchgesteckt (bis zu dem Auffangboden 6 bzw. der zweiten Lochplatte 54) und von den Eingriffsabschnitten 10 der Aufnahme-Expanderhülse 2 radial gehalten werden.

## Patentansprüche

1. Aufnahme-Expanderhülse (2) eines Werkzeugsets (100) zur steckgelagerten Aufnahme chirurgischer Werkzeuge,
mit einer Anzahl von umfangsbeabstandeten, elastisch verformbaren Klemmarmen (4), die an einer offenen Stirnseite der Aufnahme-Expanderhülse (2) eine Einstecköffnung (8) für das Einstecken eines Werkzeugs ausbilden und radial nach innen ragende Eingriffsabschnitte (10) zur kraft- und/oder formschlüssigen Kontaktierung des Werkzeugs für das Halten des Werkzeugs haben,
einem Auffangboden (6), der an einem der Einstecköffnung (8) gegenüberliegenden Endabschnitt der Aufnahme-Expanderhülse (2) ausgebildet ist und von dem sich die Klemmarme (4) aus in Axialrichtung zu der offenen Stirnseite hin erstrecken, und
Befestigungsabschnitten (14) zur form- und kraftschlüssigen Befestigung an einer eine Lochplatte (52, 54) aufweisenden Halterungsvorrichtung (50) des Werkzeugsets (100),
**dadurch gekennzeichnet, dass**
die Befestigungsabschnitte (14) angepasst und vorgesehen sind, um unmittelbar mit der Lochplatte (52, 54) zum Befestigen an der Lochplatte (52, 54) zusammenzuwirken, wobei die Befestigungsabschnitte (14) an freien, radial nach außen gebogenen oder abstehenden Endabschnitten von Befestigungsarme (12), die sich umfangsbeabstandet von dem Auffangboden (6) aus in Axialrichtung erstrecken, ausgebildet sind und dazu angepasst und vorgesehen sind, ein Loch (62) der Lochplatte (52, 54) radial außen elastisch klemmend derart zu umgreifen, dass sie das Lochplattenmaterial in Lochplattendickenrichtung formschlüssig axial hintergreifen.

2. Aufnahme-Expanderhülse (2) nach Anspruch 1, **dadurch gekennzeichnet, dass** die, vorzugsweise metallischen, Befestigungsarme (12) in Umfangsrichtung der Aufnahme-Expanderhülse (2) abwechselnd mit den Klemmarmen (4) angeordnet sind.

3. Aufnahme-Expanderhülse (2) nach Anspruch 2, **dadurch gekennzeichnet, dass** sich die Befestigungsarme (12) vom Auffangboden (6) aus in die gleiche Richtung wie die Klemmarme (4) erstrecken oder in eine entgegengesetzte Richtung gegenläufig zu den Klemmarmen (4) erstrecken.

4. Aufnahme-Expanderhülse (2) nach Anspruch 2 oder 3, **dadurch gekennzeichnet, dass** die Befestigungsabschnitte (14) an radial nach außen gebogenen oder abstehenden Abschnitten der Befestigungsarme (12) ausgebildet sind und dazu angepasst und vorgesehen sind, ein Loch (58) der Lochplatte (52) zu durchgreifen, so dass sie von radial innen elastisch klemmend das Lochplattenmaterial in Lochplattendickenrichtung hintergreifen.

5. Aufnahme-Expanderhülse (2) nach Anspruch 1, **dadurch gekennzeichnet, dass** die Klemmarme (4) an einem ersten Axialabschnitt die Eingriffsabschnitte (10) ausbilden und an einem sich in Axialrichtung unmittelbar daran anschließenden zweiten Axialabschnitt, der sich gegenüber dem ersten Axialabschnitt radial außerhalb befindet, die Befestigungsabschnitte (14) ausbilden.

6. Aufnahme-Expanderhülse (2) nach Anspruch 5, **dadurch gekennzeichnet, dass** der zweite Axialabschnitt an einem radial nach innen gebogenen, etwa U-förmigen Abschnitt, des Klemmarms (4) ausgebildet ist und dazu angepasst und vorgesehen ist, ein Loch (58, 62) der Lochplatte (52, 54) zu durchgreifen, so dass er von radial innen klemmend das Lochplattenmaterial in Lochplattendickenrichtung hintergreift.

7. Aufnahme-Expanderhülse (2) nach Anspruch 5 oder 6, **dadurch gekennzeichnet, dass** der zweite Axialabschnitt an einem freien Endabschnitt des Klemmarms (4) ausgebildet ist oder dass der zweite Axialabschnitt zwischen dem Auffangboden (6) und dem ersten Axialabschnitt des Klemmarms (4) ausgebildet ist.

8. Aufnahme-Expanderhülse (2) nach einem der Ansprüche 1 bis 7, **dadurch gekennzeichnet, dass** die Klemmarme (4), der Auffangboden (6) und die Befestigungsabschnitte (14) einstückig aus einem biegeelastischen Metall, vorzugsweise einem Federstahl, beispielsweise aus dem Werkstoff 1.4310, ausgebildet sind.

9. Aufnahme-Expanderhülse (2) nach einem der Ansprüche 1 bis 8, **dadurch gekennzeichnet, dass** die Aufnahme-Expanderhülse (2) als ein Biegeumformteil ausgebildet ist.

10. Werkzeugset (100) zur steckgelagerten Aufnahme chirurgischer Werkzeuge,
mit einer Aufnahme-Expanderhülse (2) nach einem der Ansprüche 1 bis 9, 11 und
einer Halterungsvorrichtung (50), die eine erste Lochplatte (52) mit ersten vorzugsweise runden Löchern (58) zum Einstecken eines Werkzeugs sowie eine parallel dazu beabstandete mit der ersten Lochplatte (52) verbundene zweite Lochplatte (54) mit zweiten Löchern (62) aufweist,
**dadurch gekennzeichnet, dass**
die Aufnahme-Expanderhülse (2) derart an der Haltevorrichtung (50) form- und kraftschlüssig befestigt oder befestigbar ist, dass die Befestigungsabschnitte (14) die erste Lochplatte (52) und/oder die zweite Lochplatte (54) radial klemmend derart umgreifen, dass sie das Lochplattenmaterial in Lochplattendickenrichtung formschlüssig axial hintergreifen, wobei zumindest eines der ersten Löcher (58) und/oder der zweiten Löcher (62) radial nach innen ragende Trennstege (68, 74) hat, die eine formschlüssige Verdrehsicherung für die Befestigungsabschnitte (14) bilden.

11. Werkzeugset (100) nach Anspruch 10, **dadurch gekennzeichnet, dass** die Trennstege (68, 74) derart ausgebildet sind, insbesondere jeweils zwei Trennstege (68, 74) derart miteinander verbunden sind, dass sie, insbesondere zusammen mit dem Außendurchmesser des Lochs (58, 62), mehrere im Wesentlichen dreieckige oder kreissektorförmige Befestigungsabschnittsperforationen (70, 76) bilden, durch die jeweils ein Befestigungsabschnitt (14) durchführbar ist, und/oder eine sternförmige Klemmarmperforation (76) bilden, die vorzugsweise sich von der Lochmitte sternförmig radial nach außen erstreckende Ausweichspalte hat, deren Breite größer als die Klemmarmbreite ist, so dass die Klemmarme (4) innerhalb der Klemmarmperforation (76), vorzugsweise durch die Trennstege (68) geführt, elastisch in Radialrichtung verlagerbar sind.

## Claims

1. An expander holder (2) of a tool set (100) for plug-in holding of surgical tools, comprising
a number of circumferentially spaced, elastically deformable clamping arms (4) which form an insertion opening (8) for the insertion of a tool on an open front side of the expander holder (2) and have radially inwardly projecting engagement portions (10) for the force-fitting and/or form-fitting contacting of the tool for holding the tool,
a catching base (6) formed at an end portion of the expander holder (2) opposite to the insertion opening (8) and from which the clamping arms (4) extend in the axial direction toward the open front side, and
fastening portions (14) for form-fitting and force-fitting fastening to a retaining device (50) of the tool set (100) having a perforated plate (52, 54),
**characterized in that**
the fastening portions (14) are adapted and provided to cooperate directly with the perforated plate (52, 54) for fastening to the perforated plate (52, 54), wherein the fastening portions (14) are formed on free, radially outwardly bent or protruding end portions of fastening arms (12), which extend circumferentially spaced in the axial direction from the catching base (6), and wherein the fastening portions (14) are adapted and provided and to grip around a hole (62) of the perforated plate (52, 54) in a radially clamping manner from radially outside such that they axially engage behind the perforated plate material in the thickness direction of the perforated plate in a form-fitting manner.

2. The expander holder (2) according to claim 1, **characterized in that**
the, preferably metallic, fastening arms (12) are arranged in the circumferential direction of the expander holder (2) alternately with the clamping arms (4).

3. The expander holder (2) according to claim 2, **characterized in that** the fastening arms (12) extend from the catching base (6) in the same direction as the clamping arms (4) or in an opposite direction to the clamping arms (4).

4. The expander holder (2) according to claim 2 or 3, **characterized in that** the fastening portions (14) are formed on radially outwardly bent or protruding portions of the fastening arms (12) and are adapted and provided to pass through a hole (58) of the perforated plate (52) so as to resiliently clamply engage from radially inwardly behind the perforated plate material in thickness direction of the perforated plate.

5. The expander holder (2) according to claim 1, **characterized in that** the clamping arms (4) form the engagement portions (10) at a first axial portion and form the fastening portions (14) at a second axial portion which immediately adjacent to the first axial portion in the axial direction and is located radially outside the first axial portion.

6. The expander holder (2) according to claim 5, **characterized in that** the second axial portion is formed on a radially inwardly bent, approximately U-shaped portion, of the clamping arm (4) and is adapted and provided to pass through a hole (58, 62) of the perforated plate (52, 54) so as to clamply engage from radially inside the perforated plate material in thickness direction of the perforated plate.

7. The expander holder (2) according to claim 5 or 6, **characterized in that** the second axial portion is formed at a free end portion of the clamping arm (4) or that the second axial portion is formed between the catching base (6) and the first axial portion of the clamping arm (4).

8. The expander holder (2) according to one of claims 1 to 7, **characterized in that** the clamping arms (4), the catching base (6) and the fastening portions (14) are formed in one piece from a bending elastic metal, preferably a spring steel, for example from the material 1.4310.

9. The expander holder (2) according to one of claims 1 to 8, **characterized in that** the expander holder (2) is designed as a bending forming part.

10. A tool set (100) for plug-in holding of surgical tools, comprising
an expander holder (2) according to any one of claims 1 to 9, and
a retaining device (50) having a first perforated plate (52) with first preferably round holes (58) for insertion of a tool and a second perforated plate (54) with second holes (62) connected to the first perforated plate (52) and spaced parallel thereto,
**characterized in that**
the expander holder (2) is fastened or fastenable to the retaining device (50) in a form-fitting and force-fitting manner in such a way that the fastening portions (14) grip around the first perforated plate (52) and/or the second perforated plate (54) in a radially clamping manner such that they axially engage behind the perforated plate material in the thickness direction of the perforated plate in a form-fitting manner, wherein at least one of the first holes (58) and/or the second holes (62) has radially inwardly projecting separation crosspieces (68, 74) that form a form-fitting anti-turn device for the fastening portions (14).

11. The tool set (100) according to claim 10, **characterized in that** the separation crosspieces (68, 74) are formed in such a way, in particular two separation crosspieces (68, 74) in each case are connected to one another in such a way that they form, in particular together with the outer diameter of the hole (58, 62), a plurality of essentially triangular or circular sector-shaped fastening portion perforations (70, 76) through each of which a fastening portion (14) can be passed, and/or a star-shaped clamping arm perforation (76), which preferably has avoidance gaps extending radially outwards in a star shape from the hole center, the width of which avoidance gaps is greater than the clamping arm width, so that the clamping arms (4) can be displaced elastically in the radial direction within the clamping arm perforation (76), preferably guided by the separation crosspieces (68).

## Revendications

1. Douille expansible de réception (2) d'un jeu d'instruments (100) pour la réception enfichée d'instruments chirurgicaux,
avec un certain nombre de bras de serrage (4) déformables élastiquement et espacés sur la circonférence, qui forment sur un côté avant ouvert de la douille expansible de réception (2) une ouverture d'insertion (8) pour l'insertion d'un outil et qui présentent des sections d'engagement (10) faisant saillie radialement vers l'intérieur pour la mise en contact à force et/ou par complémentarité de formes de l'outil pour le maintien de l'outil,
un fond de réception (6) qui est réalisé sur une section d'extrémité de la douille expansible de réception (2) opposée à l'ouverture d'insertion (8) et à partir duquel les bras de serrage (4) s'étendent dans le sens axial vers le côté avant ouvert, et
des parties de fixation (14) pour la fixation par complémentarité de formes et à force sur un dispositif de maintien (50) de l'ensemble d'outils (100) présentant une plaque perforée (52, 54),
**caractérisé en ce que**
les parties de fixation (14) sont adaptées et prévues pour coopérer directement avec la plaque perforée (52, 54) pour la fixation à la plaque perforée (52, 54), dans lequel les parties de fixation (14) sont fixées à des sections d'extrémité libres, pliées ou faisant saillie radialement vers l'extérieur, de bras de fixation (12), qui s'étendent dans le sens axial à une certaine distance circonférentielle du fond de réception (6), et qui sont adaptés et prévus pour entourer un trou (62) de la plaque perforée (52, 54) en le serrant de manière élastique radialement vers l'extérieur de sorte qu'ils viennent en prise par complémentarité de forme axialement derrière le matériau de la plaque perforée dans le sens de l'épaisseur de la plaque perforée.

2. Douille expansible de réception (2) selon la revendication 1, **caractérisé en ce que** les bras de fixation (12), de préférence métalliques, sont agencés en alternance avec les bras de serrage (4) dans le sens circonférentiel de la douille expansible de réception (2).

3. Douille expansible de réception (2) selon la revendication 2, **caractérisé en ce que** les bras de fixation (12) s'étendent depuis le fond de réception (6) dans le même sens que les bras de serrage (4) ou s'étendent dans un sens opposé, en sens inverse des bras de serrage (4).

4. Douille expansible de réception (2) selon la revendication 2 ou 3, **caractérisé en ce que** les parties de fixation (14) sont réalisées sur des parties des bras de fixation (12) pliées ou faisant saillie radialement vers l'extérieur et sont adaptées et prévues pour passer à travers un trou (58) de la plaque perforée (52), de sorte qu'elles viennent en prise derrière le matériau de la plaque perforée dans le sens de l'épaisseur de la plaque perforée en le serrant de manière élastique depuis l'intérieur dans le sens radial.

5. Douille expansible de réception (2) selon la revendication 1, **caractérisé en ce que** les bras de serrage (4) réalisent les sections d'engagement (10) sur une première partie axiale et réalisent les parties de fixation (14) sur une seconde partie axiale qui s'y rattache directement dans le sens axial et qui se trouve radialement à l'extérieur par rapport à la première partie axiale.

6. Douille expansible de réception (2) selon la revendication 5, **caractérisé en ce que** la seconde partie axiale est réalisée sur une partie incurvée radialement vers l'intérieur, approximativement en forme de U, du bras de serrage (4) et est adaptée et prévue pour passer à travers un trou (58, 62) de la plaque perforée (52, 54), de sorte qu'il vient en prise derrière le matériau de la plaque perforée dans le sens de l'épaisseur de la plaque perforée en le serrant radialement vers l'intérieur.

7. Douille expansible de réception (2) selon la revendication 5 ou 6, **caractérisé en ce que** la seconde partie axiale est réalisée sur une section d'extrémité libre du bras de serrage (4) ou **en ce que** la seconde partie axiale est réalisée entre le fond de réception (6) et la première partie axiale du bras de serrage (4).

8. Douille expansible de réception (2) selon l'une des revendications 1 à 7, **caractérisé en ce que** les bras de serrage (4), le fond de réception (6) et les parties de fixation (14) sont formés d'un seul tenant à partir d'un métal pouvant être fléchi élastiquement, de préférence un acier à ressort, par exemple à partir du matériau 1.4310.

9. Douille expansible de réception (2) selon l'une quelconque des revendications 1 à 8, **caractérisé en ce que** la douille expansible de réception (2) est réalisée sous la forme d'une pièce déformée par flexion.

10. Ensemble d'outils (100) pour la réception enfichée d'outils chirurgicaux,
avec une douille expansible de réception (2) selon l'une quelconque des revendications 1 à 9, et
un dispositif de maintien (50) qui présente une première plaque perforée (52) avec des premiers trous (58) de préférence ronds pour l'insertion d'un outil, ainsi qu'une seconde plaque perforée (54) avec des seconds trous (62) reliée parallèlement à la première plaque perforée (52) et espacée de celle-ci,
**caractérisé en ce que**
la douille expansible de réception (2) est fixée ou peut être fixée au dispositif de maintien (50) par complémentarité de formes et à force de sorte que les parties de fixation (14) entourent la première plaque perforée (52) et/ou la seconde plaque perforée (54) en les serrant radialement, de sorte qu'elles viennent en prise axialement derrière et par complémentarité de forme avec le matériau de la plaque perforée dans le sens de l'épaisseur de la plaque perforée, dans lequel au moins l'un des premiers trous (58) et/ou des seconds trous (62) présente des entretoises de séparation (68, 74) faisant saillie radialement vers l'intérieur et qui forment une sécurité antirotation par complémentarité de forme pour les parties de fixation (14).

11. Jeu d'instruments (100) selon la revendication 10, **caractérisé en ce que** les entretoises de séparation (68, 74) sont réalisées de sorte, en particulier respectivement deux entretoises de séparation (68, 74) sont reliées entre elles de sorte qu'elles forment, en particulier avec le diamètre extérieur du trou (58, 62), plusieurs perforations de partie de fixation (70, 76) sensiblement triangulaires ou en forme de secteur de cercle, à travers lesquelles il est possible de faire passer respectivement une partie de fixation (14), et/ou forment une perforation de bras de serrage (76) en forme d'étoile, qui présente de préférence une fente d'évitement s'étendant radialement vers l'extérieur en forme d'étoile à partir du centre du trou, dont la largeur est supérieure à la largeur du bras de serrage, de sorte que les bras de serrage (4) puissent être déplacés élastiquement dans le sens radial à l'intérieur de la perforation de bras de serrage (76), de préférence guidés à travers les entretoises de séparation (68).
